# EUROPEAN PATENT APPLICATION

(11) **EP 4 560 304 A1**
(43) Date of publication of application: **28.05.2025**
(21) Application number: 23842940.1
(22) Date of filing: 14.07.2023
(51) Int. Cl.: G01N 27/327, C08F 220/38, G01N 27/416

(54) **REAGENT LAYER AND BIOSENSOR COMPRISING REAGENT LAYER**

(30) Priority: 21.07.2022 JP 2022116519; 21.07.2022 JP 2022116522
(71) Applicant: PHC Holdings Corporation, Tokyo 100-8403 (JP)
(72) Inventor: NAKATO, Junya, Ehime 791-0395 (JP); HANEDA, Keigo, Ehime 791-0395 (JP); YANO, Setsuko, Ehime 791-0395 (JP)
(74) Representative: Eisenführ Speiser
(86) International application number: PCT/JP2023/026089
(87) International publication number: WO 2024/019017

(57) **Abstract**

In an embodiment of the present invention, there is provided a reagent layer comprising a polymer containing a proton accepting group in a repeating unit and having a pH buffering ability, and an oxidoreductase that oxidizes or dehydrogenates an analyte.

## Description

### TECHNICAL FIELD

The present invention relates to a reagent layer and a biosensor including the reagent layer.

### BACKGROUND ART

In recent years, in various fields such as the medical field, a biosensor can be used to measure an analyte in a sample (cell culture solution or the like). As a method for measuring an analyte, for example, an electrochemical measurement method can be used.

As a biosensor in an electrochemical measurement method, there is a biosensor including an electrode (working electrode, counter electrode, reference electrode) and an enzyme membrane provided on the electrode (see Patent Document 1). The biosensor including the electrode and the enzyme membrane can be disposed in a weighing unit having a structure that enables continuous supply of a buffer solution having a pH buffering ability and sample injection into the buffer solution. Various analytes can be measured by appropriately selecting an enzyme on which an analyte to be measured acts as a substrate.

### PRIOR ART DOCUMENT

### PATENT DOCUMENT

Patent Document 1: JP-A-10-78405

### SUMMARY OF THE INVENTION

### PROBLEMS TO BE SOLVED BY THE INVENTION

Here, the inventors of the present application have newly found that there are matters that can be improved with respect to the configuration of the conventional biosensor. Specifically, prior biosensors may be disposed in a structure capable of continuous feed of a buffer solution and sample injection into the same buffer solution. However, such a conventional biosensor is difficult to cope with a method of continuously and directly monitoring a sample without continuous feed of a buffer solution. Therefore, it is desired that a reagent layer (corresponding to the enzyme membrane of Patent Document 1) as a constituent of the biosensor has a property of pH buffering ability.

The present invention has been made in view of such problems. That is, an object of the present invention is to provide a reagent layer having pH buffering ability and a biosensor including the reagent layer.

### SOLUTIONS TO THE PROBLEMS

To achieve the above object, in an embodiment of the present invention,
there is provided a reagent layer including a polymer containing a proton accepting group in a repeating unit and having a pH buffering ability, and an oxidoreductase that oxidizes or dehydrogenates an analyte.

In an embodiment of the present invention, a biosensor including the reagent layer is provided.

### EFFECTS OF THE INVENTION

According to an embodiment of the present invention, the reagent layer itself can have the property of pH buffering ability.

### BRIEF DESCRIPTION OF THE DRAWINGS

[Fig. 1] Fig. 1 is a cross-sectional view schematically showing a configuration of a reagent layer containing a reagent according to an embodiment of the present invention.
[Fig. 2] Fig. 2 is a cross-sectional view schematically illustrating a main configuration of a biosensor according to an embodiment of the present invention.
[Fig. 3] Fig. 3 is a cross-sectional view schematically illustrating an action mechanism provided based on a main configuration of a biosensor according to an embodiment of the present invention.
[Fig. 4] Fig. 4 is a cross-sectional view schematically illustrating an action mechanism provided based on a main configuration of a biosensor according to another embodiment of the present invention.
[Fig. 5] Fig. 5 is a graph showing a relation between a measurement elapsed time and a current response value in Example 1A.
[Fig. 6] Fig. 6 is a graph showing a relation between a measurement elapsed time and a current response value in Comparative Example 1A.
[Fig. 7] Fig. 7 is a graph showing a relation between a measurement elapsed time and a current response value in Example 2A.
[Fig. 8] Fig. 8 is a graph showing a relation between a measurement elapsed time and a current response value in Comparative Example 2A.

### DETAILED DESCRIPTION

Hereinafter, an embodiment of the present invention (invention A) is specifically described. An overall configuration of the biosensor is described below. Subsequently, a characteristic part of the present invention is described.

### [Overall configuration of biosensor]

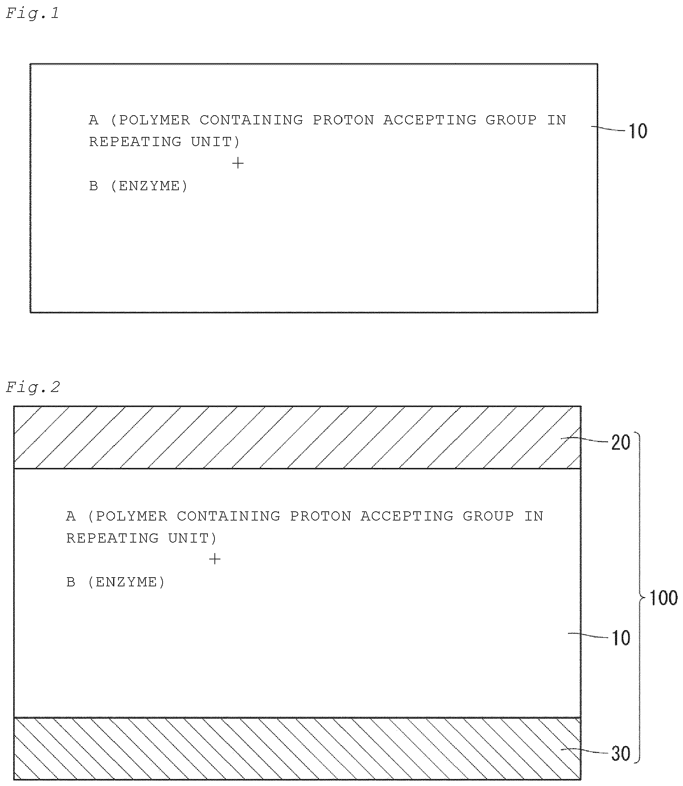

Hereinafter, an overall configuration of a biosensor according to an embodiment of the present invention is described. Fig. 1 is a cross-sectional view schematically showing a configuration of a reagent layer according to an embodiment of the present invention. Fig. 2 is a cross-sectional view schematically illustrating a main configuration of the biosensor according to the embodiment of the present invention.

A biosensor 100 according to an embodiment of the present invention includes a working electrode 30 located on a surface of a substrate, a reagent layer 10 located on a surface of the working electrode, and a protective membrane 20 located on a surface of the reagent layer 10 (see Figs. 1 and 2). In the present embodiment, for the sake of convenience, the working electrode 30 as a constituent of the electrode part is illustrated, and illustration of the counter electrode and the reference electrode as other constituents of the electrode part is omitted.

The "biosensor" as used herein is a measurement device that converts a chemical change generated by a molecular recognition reaction between a substrate (corresponding to an analyte) and an enzyme (corresponding to a receptor) into an electric signal using a combination of an enzyme-substrate and the like, and measures the metabolic rate, concentration, and the like of the analyte according to the intensity of the obtained electric signal.

As the substrate, an insulating substrate can be used. Although not particularly limited, the substrate can be made of a material such as polyethylene terephthalate, polyamide, or polyimide having a thickness of several hundred µm. In one embodiment, the electrode part of the biosensor 100 can be inserted into a liquid sample (cell culture solution or the like). After the biosensor 100 is inserted, a voltage can be applied to the electrode part.

As is specifically described below, the reagent layer 10 may contain at least an enzyme B (see Fig. 1). As the enzyme B, one in which an analyte to be measured acts as a substrate can be selected. Such selection enables measurement of various analytes. The enzyme B may be an oxidoreductase that oxidizes or dehydrogenates an analyte.

Examples of the oxidoreductase include glucose oxidase, lactate oxidase, cholesterol oxidase, bilirubin oxidase, glucose dehydrogenase, lactate dehydrogenase, amino acid oxidase, amino acid dehydrogenase, glutamate oxidase, glutamate dehydrogenase, fructosylamino acid oxidase, fructosyl peptide oxidase, 3-hydroxybutyrate dehydrogenase, alcohol oxidase, and/or alcohol dehydrogenase.

The reagent layer may further contain a mediator and/or conductive particles. Examples of the conductive fine particles include carbon black and carbon nanotubes. The term "mediator" as used herein means, in a broad sense, an oxidation-reduction substance that mediates electron transfer, and in a narrow sense, a substance that is responsible for transfer of electrons generated by an oxidation-reduction reaction of an analyte in the following biosensor.

Although not particularly limited, examples of the mediator include a metal complex (for example, an osmium complex, a ruthenium complex, an iron complex, or the like), a quinone compound (examples thereof include benzoquinone, naphthoquinone, phenanthrenequinone, phenanthroline quinone, anthraquinone, and derivatives thereof), a phenazine compound, a viologen compound, a phenothiazine compound, and a phenol compound.

More specifically, As the mediator, one or more selected from the group consisting of phenazine derivatives such as potassium ferricyanide, hexaammine ruthenium, ferrocene, poly(1-vinylimidazole)-bis(bipyridine)chloroosmium, hydroquinone, 2-methyl-1,4-benzoquinone, 1,2-naphthoquinone-4-sulfonate, 9,10-phenanthrenequinone-2-sulfonate, 9,10-phenanthrenequinone-2,7-disulfonate, 1,10-phenanthroline-5,6-dione, anthraquinone-2-sulfonate, 1-methoxy-5-methylphenazinium methylsulfate and 1-methoxy-5-ethylphenazinium ethylsulfate, methylviologen, benzylviologen, methylene blue, methylene green, 2-aminophenol, 2-amino-4-methylphenol, and, 2,4-diaminophenol are used.

The protective membrane 20 can be configured to cover the reagent layer 10 and the electrode part. The protective membrane 20 is disposed so as to be able to allow the analyte to permeate to the working electrode side while controlling the permeation rate of the analyte (lactic acid or the like) of the cells in the culture medium. In addition, the protective membrane 20 is disposed so as to be able to suppress the components contained in the reagent layer 10 on the working electrode from flowing out to the outside of the protective membrane 20. That is, the "protective membrane" used in the present specification is a membrane that contributes to suppression of leakage of a substance contained in the reagent layer to the outside of the protective membrane, and is a membrane having a hole through which an analyte present outside the protective membrane is movable to the reagent layer side via the protective membrane.

The protective membrane desirably contains a biocompatible polymer. The protective membrane can be obtained, for example, by dissolving a polymer and a crosslinking agent in an alcohol solvent, for example, a buffer solution-alcohol containing solvent to form a membrane solution, applying the membrane solution to a reagent layer, drying the membrane solution, or immersing a laminate of an electrode and a reagent layer in the membrane solution, pulling up the laminate, and drying the laminate.

The polymer contained in the protective membrane may include a polymer having a biocompatible phosphorylcholine group and a methacryloyl group or an acryloyl group as a polymerizable group. As an example, a 2-methacryloyloxyethyl phosphorylcholine (MPC) polymer may be included.

In addition, as the polymer contained in the protective membrane, t-butyl acrylate having a heterocyclic nitrogen group and the like can be contained. As the heterocyclic nitrogen group, for example, a pyridyl group or the like can be selected. As an example, a random copolymer of styrene-2-vinylpyridine-terbutyl methacrylate (S2VPtBuMA), a random copolymer of tripropylene glycol methyl ether methacrylate-styrene-4-vinylpyridine (TGMAS4VP), and/or poly(ter.butyl methacrylate-b-4-vinylpyridine (tBuMA4VP)) may be selected.

As the crosslinking agent, a crosslinking agent having a reactive group capable of reacting with the heterocyclic nitrogen group can be used. An example is poly(ethylene glycol) diglycidyl ether.

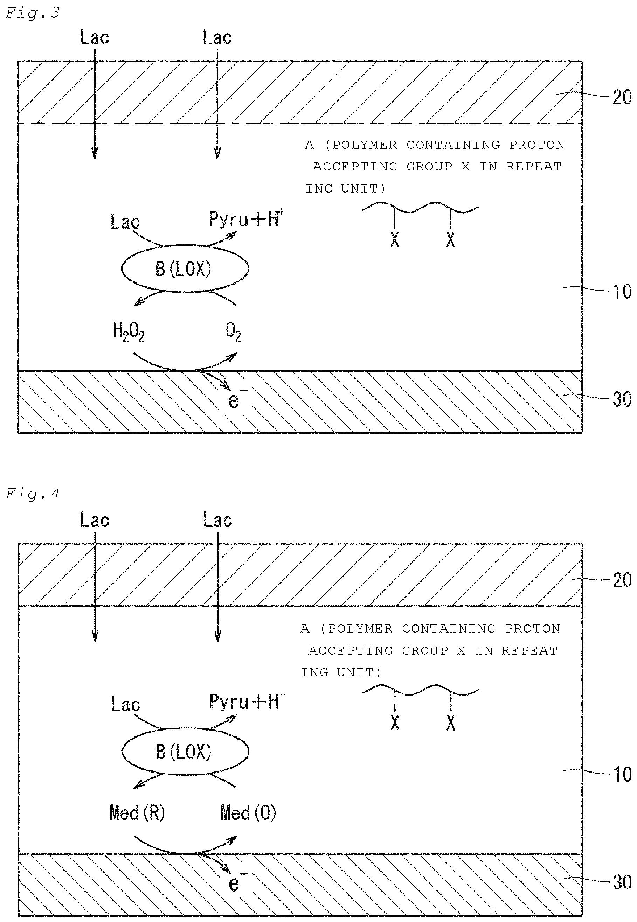

Hereinafter, a mechanism of a reaction action provided based on biosensor 100 including the above constituents is described. Fig. 3 is a cross-sectional view schematically illustrating an action mechanism provided based on a main configuration of a biosensor according to an embodiment of the present invention. Fig. 4 is a cross-sectional view schematically illustrating an action mechanism provided based on a main configuration of a biosensor according to another embodiment of the present invention.

In one example of the present invention, the analyte that moves from the culture medium to the reagent layer 10 via the protective membrane 20 in a state where the electrode part is immersed in the culture medium is oxidized in the reagent layer 10 using the enzyme B as a catalyst and dissolved oxygen. The concentration of the analyte can be measured by electrically detecting hydrogen peroxide generated at that time.

Taking the case of detecting lactic acid as an example, lactic acid (corresponding to Lac in Fig. 3) that has moved from the culture medium to the reagent layer 10 through the protective membrane 20 is oxidized by an enzymatic reaction with an enzyme of the reagent layer 10 (corresponding to lactate oxidase (LOx) in Fig. 3). Such oxidation can result in the formation of pyruvic acid (corresponding to Pyru in Fig. 3), hydrogen peroxide, and a proton in an ionization state (H+). The concentration of lactic acid can be measured by electrically measuring hydrogen peroxide generated during the formation. In this case, the biosensor 100 can function as a lactic acid sensor.

As shown in Fig. 4, the reagent layer 10 may contain a mediator. As described above, the mediator is an oxidation-reduction substance that mediates transmission of electrons generated by an oxidation-reduction reaction of an analyte (corresponding to lactic acid) in biosensor 100. Therefore, due to the presence of the mediator, the generated electrons can be suitably transmitted.

### [Characteristic part (reagent layer) of the present invention]

Hereinafter, characteristic parts of the present invention are described. The present invention is characterized by a configuration of reagent layer 10 which is a constituent of a biosensor (see Fig. 1).

The present inventors have intensively studied a configuration for continuously and directly monitoring an analyte present in a cell culture solution or the like without continuously supplying a buffer solution having a pH buffering ability as in a conventional biosensor. As a result, the inventor of the present application has devised a configuration in which the reagent layer 10, which is a constituent of the biosensor 100, contains the polymer A having pH buffering ability together with "containing a proton accepting group in a repeating unit " in addition to the above-described enzyme B. The term "proton accepting group" as used herein refers to a functional group capable of accepting protons (H⁺) contained in a repeating unit of a polymer.

When the repeating unit of the polymer A contains a proton accepting group, it is possible to accept a proton in an ionization state that can be generated when the analyte is oxidized by an enzymatic reaction with the enzyme B in the reagent layer 10. From the viewpoint of preventing detachment of the polymer A to the outside of the reagent layer 10, the weight average molecular weight (Mw) of the polymer A is preferably 10,000 or more.

As described above, the polymer A remains in the vicinity of the enzyme B in the reagent layer 10, and it is possible to suppress the pH of the reaction field from deviating from the optimal pH of the enzyme B. That is, the pH of the reaction field can be maintained in a certain range. Therefore, deactivation of enzyme B can be avoided, and as a result, durability of biosensor 100 can be improved. Specifically, the biosensor 100 including the reagent layer 10 having the above characteristics is directly inserted into the culture tank to which the culture medium is supplied, so that the analyte can be suitably continuously measured. The term "sensor durability" as used herein refers to a retention rate of a current response value of a sensor during a measurement period.

As the proton accepting group contained in the repeating unit of the polymer A, a heterocyclic nitrogen group can be used. For example, the heterocyclic nitrogen group can be at least one selected from the group consisting of an imidazole group, a pyridyl group, an indolyl group, a quinolyl group, an isoquinolyl group, a tetrahydroquinolyl group, a thiazole group, an indolidyl group, an imidazopyridyl group, an acridinyl group, a tetrazole group, a triazole group, a pyrazyl group, a morpholyl group, and a piperazyl group.

An example of the polymer A containing a heterocyclic nitrogen group in a repeating unit can be, for example, the following polyvinyl imidazole PolyIMZ.

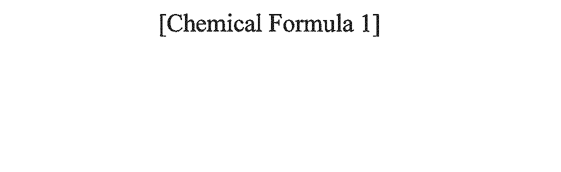

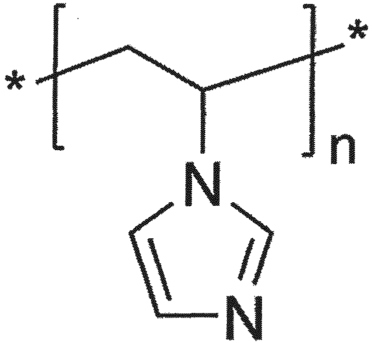

Another example of the polymer A containing a heterocyclic nitrogen group in the repeating unit can be, for example, the following poly-L-histidine (PolyH).

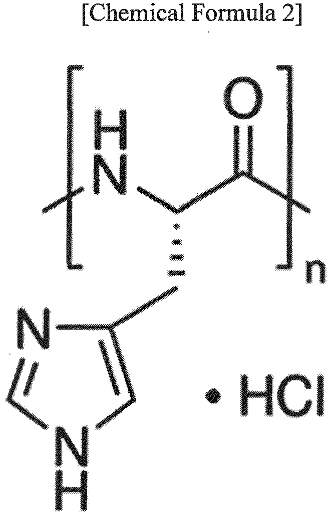

The proton accepting group contained in the repeating unit of the polymer A can be at least one selected from the group consisting of a phosphate group, a sulfo group, and a carboxy group in an ionization state. As an example of the polymer A containing the functional group in an ionization state in the repeating unit, sodium polyphosphate having a degree of polymerization of 700 to 1000 and a weight average molecular weight (Mw) of 70,000 or more and 100,000 or less can be used.

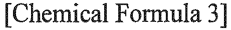

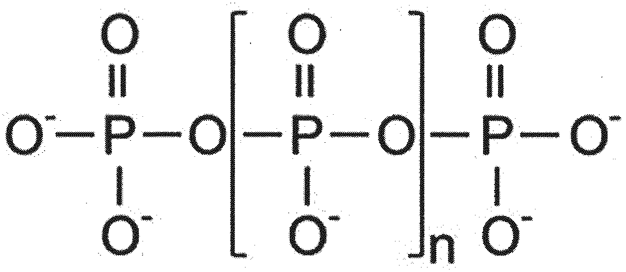

As the polymer A, a polymer containing an ionic group in addition to a proton accepting group in a repeating unit may be used. The term "ionic group" as used herein refers to an ionic functional group that is contained in a repeating unit of a polymer and is capable of providing hydrophilicity.

As an example, Polymer A may comprise a moiety wherein the repeating unit is derived from 4-vinylpyridine and a moiety derived from 3-[[2-(methacryloyloxy)ethyl]dimethylammonio]propane-1-sulfonate. That is, in the repeating unit of the polymer A, the first moiety having an ionic group may contain 3-[[2-(methacryloyloxy)ethyl]dimethylammonio]propane-1-sulfonate. In the repeating unit of the polymer A, the second moiety having a proton accepting group may contain 4-vinylpyridine. For example, the polymer A may have the following structure.

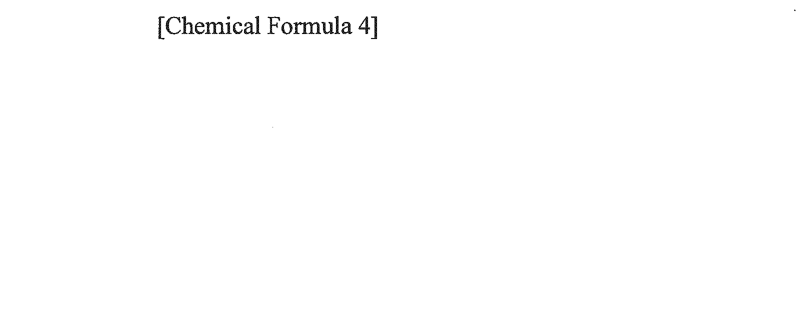

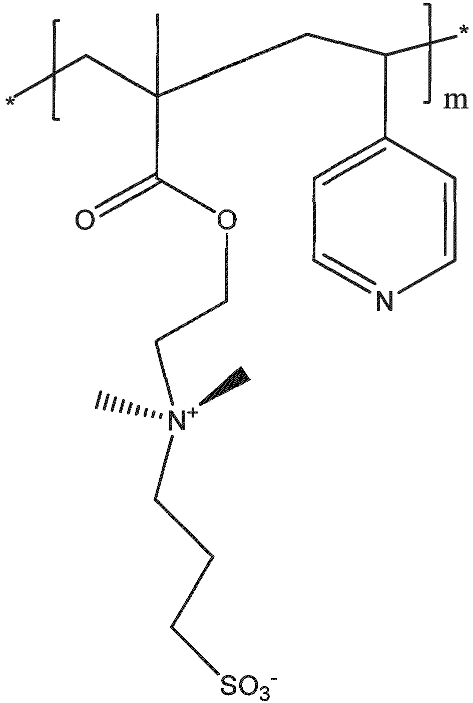

### [Method for producing biosensor]

Hereinafter, an example of a method for producing the biosensor is described.

First, an insulating substrate is prepared. After the preparation of the insulating substrate, carbon ink or the like is printed on the insulating substrate to form a conductive thin membrane constituting the working electrode 30, the counter electrode, and the wiring. In addition, an Ag/AgCl ink is printed on the conductive thin membrane to form a reference electrode.

Alternatively, a conductive thin membrane (corresponding to a working electrode) selected from metals such as gold, platinum, and palladium can also be formed by depositing the conductive thin membrane by a sputtering method, a vapor deposition method, ion plating, or the like.

The surface of the working electrode can be coated with Nafion containing a fluorocarbon as a main chain and a sulfo group as a side chain. The thickness of the conductive thin membrane may be 10 nm to several hundred nm. The counter electrode and/or the reference electrode may be disposed on the peripheral edge of the working electrode, for example, on the back side of the substrate. An insulating resist layer can be formed on parts other than these electrode forming parts.

Thereafter, a reagent constituting the reagent layer of the present invention is applied onto the working electrode. The reagent contains a polymer A and an enzyme B containing a proton accepting group in the repeating unit. In addition to these polymer A and enzyme B, the reagent can further include a suspension of conductive particles such as carbon particles for imparting conductivity and an aqueous solution of a mediator. After the application of the reagent, the reagent layer 10 can be formed by drying the reagent applied to the working electrode.

After the formation of the reagent layer 10, the polymer solution for the protective membrane 20 is applied to the surface of the reagent layer 10, and then dried at room temperature. Alternatively, the electrode 30 with the reagent layer 10 is repeatedly immersed in the polymer solution for the protective membrane 20, pulled up, and dried a plurality of times. As a result, the protective membrane 20 can be formed so as to cover the reagent layer 10 and the electrode part. As described above, biosensor 100 can be produced.

### Examples

Hereinafter, examples of the present invention (invention A) are described.

### Example 1A

### [Production of biosensor]

A biosensor was produced through the following steps (1) to (6).

### ● Step (1): Production of electrode

Using a screen printer 250IP (manufactured by Celia Corporation), carbon ink (manufactured by Fujikura Kasei Co., Ltd.) was printed on an insulating substrate to form a conductive thin membrane. The conductive thin membrane constitutes a working electrode and wiring. Subsequently, a first resist membrane for regulating the extension range of the reagent layer was laminated on the insulating substrate. Further, a water-repellent membrane for regulating the extension ranges of the cation exchange membrane and the protective membrane was attached onto the first resist membrane.

### ● Step (2): Preparation of reagent liquid

The following reagents were mixed so as to have the following final concentrations, and reacted for about 1 hour to prepare a reagent liquid.
· Sodium phosphate buffer solution (pH 6.5) final concentration: 10 mM
· Sodium chloride (manufactured by FUJIFILM Wako Pure Chemical Corporation) final concentration: 20 mM
· Final concentration of carbon dispersion liquid 5 mg/mL
· Polymer-bonded type PNT final concentration 4 (absorbance at 608 nm)
· Hydroxypropyl cellulose (NISSO HPC-VH, manufactured by Nippon Soda Co., Ltd.), final concentration: 0.05% (w/v)
· Polyvinyl imidazole final concentration 6.59 mg/mL
· Lactate oxidase (LOX T-47: manufactured by Asahi Kasei Pharma Corporation) final concentration: 400U/mL
· 25% glutaraldehyde solution (manufactured by FUJIFILM Wako Pure Chemical Corporation), final concentration: 0.002% (w/v)

A sodium phosphate buffer solution was prepared with disodium hydrogen phosphate (manufactured by FUJIFILM Wako Pure Chemical Corporation) and sodium dihydrogen phosphate (manufactured by FUJIFILM Wako Pure Chemical Corporation).

The carbon dispersion liquid was obtained by mixing Ketjen black (EC300J, manufactured by Lion Specialty Chemicals Co., Ltd.) with a 5 mg/mL hydroxypropyl cellulose (NISSO HPCL, manufactured by Nippon Soda Co., Ltd.) solution so as to have a carbon concentration of 16 mg/mL, and treating the mixture with an ultrasonic homogenizer for 3 minutes or more.

The carbon dispersion liquid was used after being treated with an ultrasonic bath for about 10 minutes before use. The concentration of the polymer-bonded type PNT was adjusted based on the value obtained by diluting the solution of the polymer-bonded type PNT 25 times, charging 100 µL of the diluted solution into a microplate, and measuring the absorption spectrum with a plate reader to confirm the concentration of the PNT in the solution. For example, the final concentration 4 is a concentration showing an absorbance of 0.16 when the polymer-bonded type PNT is diluted 25 times.

The polymer-bonded type PNT was obtained through the following steps.

### [I. Synthesis of PNT-70]

### (1) Synthesis of sulfonic acid fragment

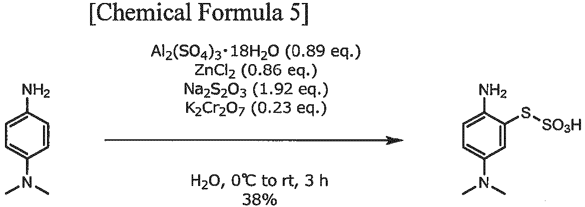

First, a sulfonic acid fragment was synthesized by the above reaction.

### (2) Synthesis of carboxylic acid fragment

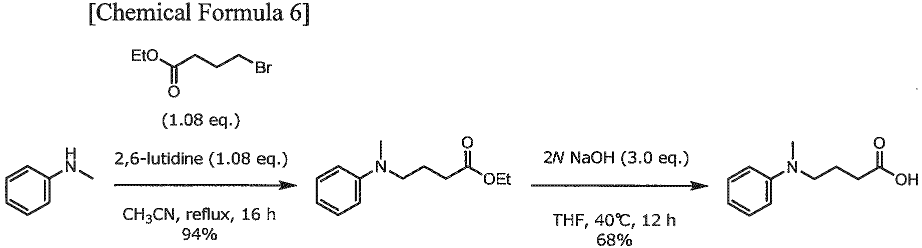

Carboxylic acid fragments were synthesized by a two-step reaction in acetonitrile and tetrahydrofuran (THF) as described above.

### (3) Synthesis of PNT-34

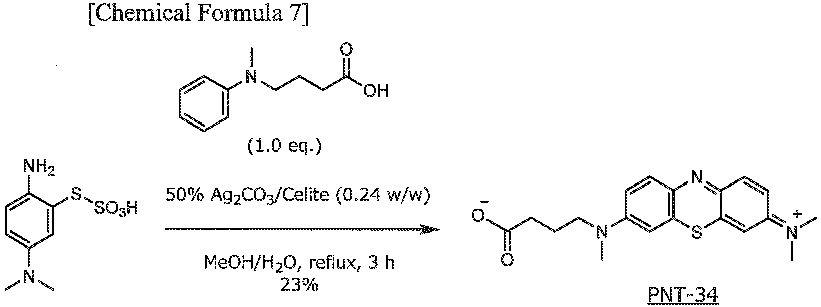

The sulfonic acid fragment and the carboxylic acid fragment synthesized above were suspended in MeOH/H₂O, and 50%Ag₂CO₃/celite was added in parts over 15 minutes at an internal temperature of about 47°C. After the addition, the mixture was stirred for 2.5 hours at an internal temperature of about 68°C. After the mixture was allowed to cool to room temperature, celite filtration was performed, and the filtrate was concentrated. The residue was purified a plurality of times by silica gel column chromatography to obtain PNT-34.

### (4) Synthesis of condensate (PNT-68)

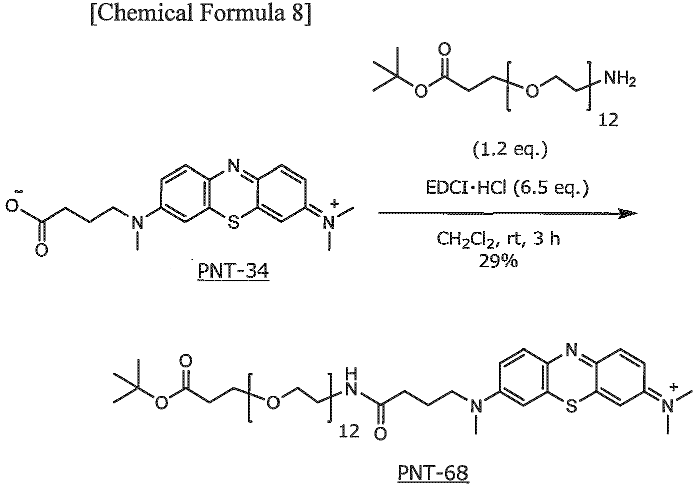

PNT-34 was dissolved in dichloromethane under an Ar atmosphere, and amino-PEG12-t-butyl ester and 1-ethyl-3-(3-dimethylaminopropyl) carbodiimide hydrochloride (EDCI·HCl) were added, then the mixture was stirred at room temperature for 3 hours. The reaction solution was concentrated, and then purified 3 times by silica gel column chromatography to obtain a condensate (PNT-68).

### (5) Synthesis of deprotected product (PNT-69)

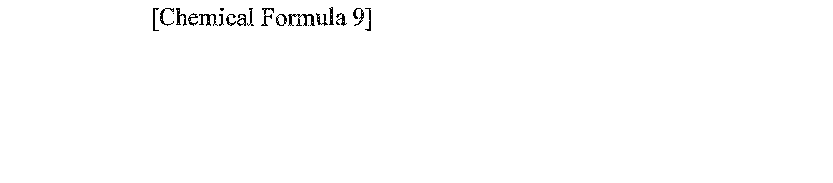

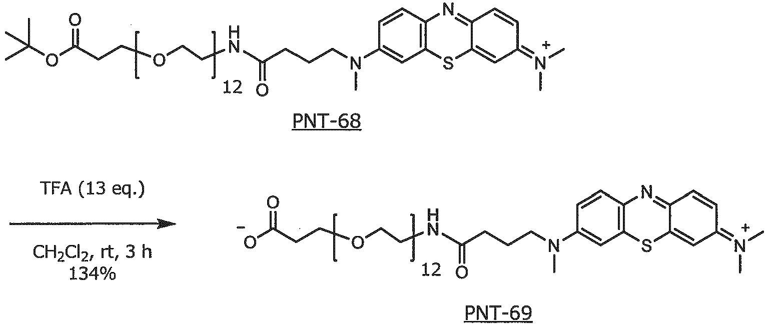

(4) The condensate (PNT-68) obtained above was dissolved in dichloromethane, trifluoroacetic acid (TFA) was added, and the mixture was stirred at room temperature for 3 hours. The reaction solution was concentrated, and then azeotroped with toluene several times to obtain a deprotected product (PNT-69).

### (6) Synthesis of PNT-69NHS form (PNT-70)

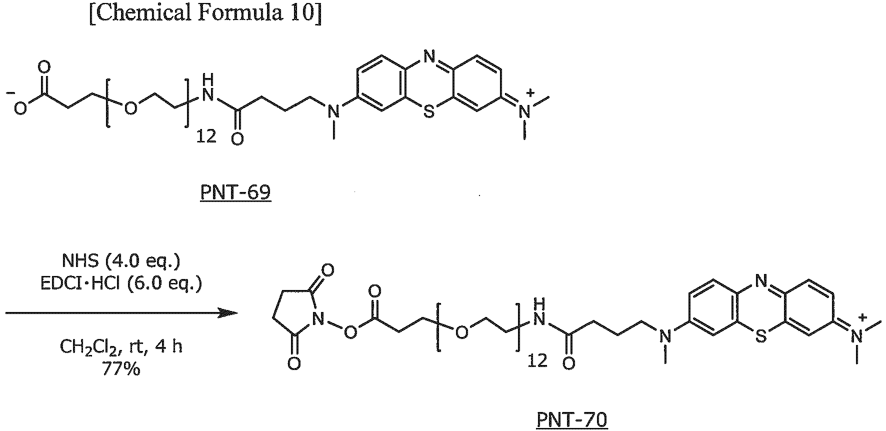

The deprotected product (PNT-69) was dissolved in dichloromethane, and N-hydroxysuccinimide (NHS) and EDCI·HCl were added, then the mixture was stirred at room temperature for 4 hours. The reaction solution was purified by silica gel column chromatography to obtain a PNT-69NHS form (PNT-70) in which the carboxy group at the terminal of the PEG chain (PEG12) was activated (NHS esterification).

### [II. Synthesis of polymer C]

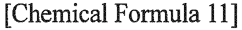

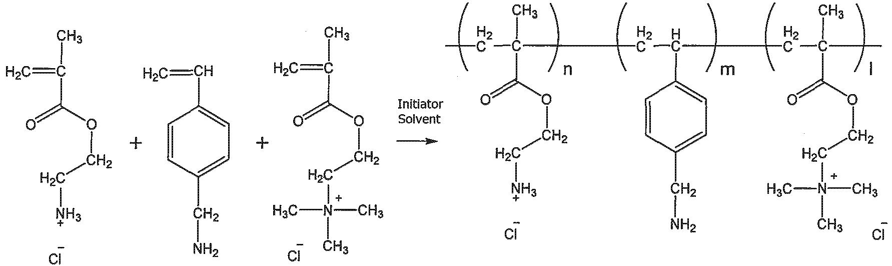

First, 2-aminoethyl methacrylate hydrochloride, (4-vinylphenyl)methanamine, and methacryloylcholine chloride were prepared. Then, 0.97 mmol of 2-aminoethyl methacrylate hydrochloride, 0.97 mmol of (4-vinylphenyl) methanamine, 11.97 mmol of methacloylcholine chloride, 0.08 mmol of V-50 and 10.49 g of ethanol were charged into a four-necked flask. A polymer C corresponds to a polymer of n : m : 1= 21.2 : 8.1 : 70.7 as a result of calculating the molar ratio from the result of 1H-NMR. In addition, as a result of measurement by gel permeation chromatography (GPC), the number average molecular weight Mn of the polymer was 37774, and the weight average molecular weight Mw was 214367.

### [Synthesis of polymer-bonded type PNT]

A PNT-69NHS form (PNT-70) was dissolved in MilliQ water to 21.05 mg/mL (liquid (1)). As a high molecular weight polymer, the polymer C was dissolved in MilliQ water to 15 mg/mL (liquid (2)). Next, WSC (DOJINDO WO01) was dissolved in MilliQ water to 20 mg/mL (liquid (3)). 40 µL of the liquid (1), 140.3 µL of the liquid (2), 383.4 µL of the liquid (3) and 96 µL of a separately prepared 250 mM 2-morpholinoethanesulfonic acid (MES) buffer solution (pH 6.0) were mixed, and the total volume was adjusted to 1200 µL with MilliQ water. Thereafter, the mixture was reacted at room temperature for about 20 hours with stirring. Thereafter, a centrifugal ultrafiltration filter (Amicon Ultra-4 50k; Merck Millipore) to collect a liquid from which a low molecular weight molecule was removed. As described above, a polymer-bonded type PNT was obtained.

### ● Step (3): Application of reagent liquid

0.9 µL of the reagent prepared in the step (2) was applied onto the electrode produced in the step (1), and dried over overnight. As a result, a reagent layer was formed on the working electrode.

### ● Step (4): Preparation of polymer solution for protective membrane

2-Methacryloyloxyethyl phosphorylcholine (MPC) polymer (Lipidure-CM5206 NOF CORPORATION) was dissolved in ethanol at a concentration of 4% (w/w). In addition, a random copolymer of styrene-2-vinylpyridine-ter.butyl methacrylate (hereinafter referred to as "S2VPtBuMA") was dissolved in 2-propanol at a concentration of 7% (w/w).

### ● Step (5): Application of protective membrane solution

Onto the electrode produced in the step (3), 1 µL of the Lipidure-CM5206 solution prepared in the step (4) was applied, and dried at room temperature. Thereafter, 1 µL of the S2VPtBuMA solution was applied and dried at room temperature. Thus, a protective membrane was formed on the reagent layer.

### ● Step (6): Formation of electrode part

The biosensor electrode produced in the step (5) was used as a working electrode, and a gold electrode as a counter electrode and an Ag/AgCl electrode (saturated KCl) (produced by BAS Inc.) as a reference electrode were combined to produce a three-electrode type electrode part.

### Comparative Example 1A

Comparative Example 1A is different from Example 1A in that polyvinylimidazole is not added in the step (2): preparation of a reagent liquid. The other points are the same as those in Example 1A, and thus description thereof is omitted in order to avoid duplication of description.

### [Electrochemical measurement, etc. using biosensor]

Next, electrochemical measurement was performed using the biosensors produced in Comparative Example 1A and Example 1A. In addition, the pH of the measurement liquid at the time of adding each concentration of lactic acid in Comparative Example 1A was measured.

In the electrochemical measurement using the biosensor, a change in the current value when lactic acid was added after an elapse of a predetermined time in an RPMI culture medium heated to about 37°C was measured by an amperometric method using a potentiostat (manufactured by BAS Inc.).

As an RPMI culture medium, RPMI-1640 Medium (R1383 manufactured by Sigma-Aldrich) was used, and in order to simulate buffering ability in a CO₂ incubator, MES(2-Morpholinoethanesulfonic acid monohydrate) (manufactured by DOJINDO LABORATORIES) and MOPS (3-Morpholinopropanesulfonic acid) (manufactured by DOJINDO LABORATORIES) were added as buffer solution components so as to have a final concentration of 25 mM, and the pH was adjusted to 7.4.

Specifically, the current response value of the sensor obtained when the lactic acid solution was added to the culture medium so as to have final concentrations of 10 mM, 20 mM, 30 mM, and 40 mM was continuously measured every 500 seconds from 1000 seconds after the start of measurement. As a lactic acid solution to be added, a solution obtained by diluting L-lactic acid (manufactured by Tokyo Chemical Industry Co., Ltd.) to 0.5M with a 0.5M MOPS solution was used.

After completion of the measurement, the sensor was immersed in RPMI culture medium and stored at 37°C. Then, in order to evaluate the durability of the lactic acid sensor, the measurement of the current response value was performed before storage of the sensor, 7 days after storage, and 13 days after storage, respectively.

### [Measurement results]

- pH value of measurement liquid at the time of addition of each concentration of lactic acid in Comparative Example 1A

The pH of the measurement liquid at the time of adding each concentration of lactic acid in Comparative Example 1A was as follows.

From the results in the following Table 1, from the results in the following Table 1, it was found that as the lactic acid concentration in the measurement liquid increases, the pH of the measurement liquid may decrease to deviate from the optimal pH (6 to 8) in some cases.

**(Table 1)**

| | | | | | |
|---|---|---|---|---|---|
| Lactic acid concentration (mM) | 0 | 10 | 20 | 30 | 40 |
| pH of measurement liquid | 7.35 | 6.84 | 6.45 | 6.05 | 5.53 |

### ● Relation between measurement elapsed time and current response value in each of Example 1A and Comparative Example 1A

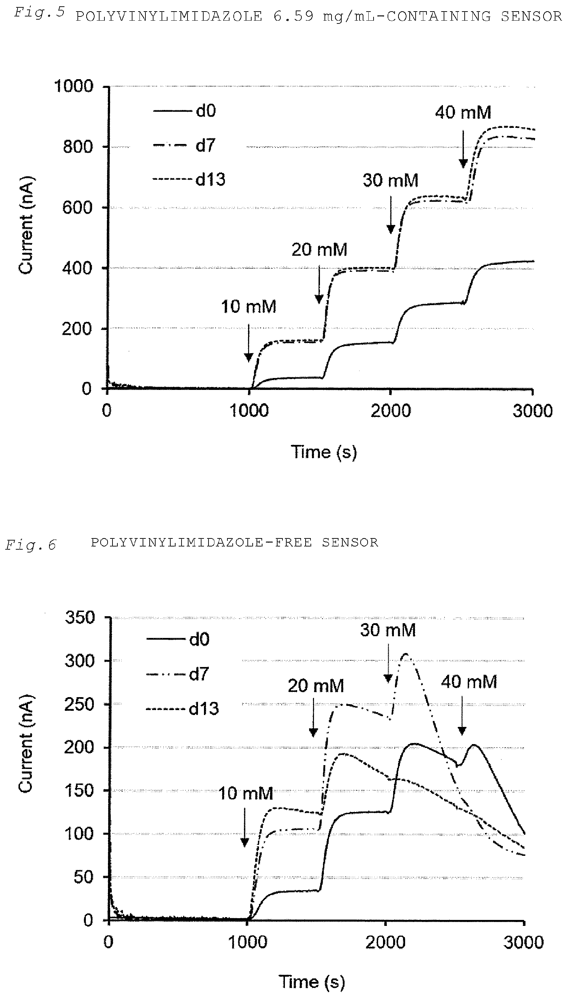

The relation between the measurement elapsed time and the current response value in Example 1A is shown in Fig. 5, and the relation between the measurement elapsed time and the current response value in Comparative Example 1A is shown in Fig. 6.

### [Sensor responsiveness]

As shown in Figs. 5 and 6, it was found that in Example 1A in which polyvinylimidazole was added to the sensor reagent liquid, the current response value of the sensor to lactic acid at each concentration (10 mM to 40 mM) increased stepwise, and the current value after addition of lactic acid was kept constant, as compared with Comparative Example 1A in which polyvinylimidazole was not added.

On the other hand, in Comparative Example 1A, it was found that the current response value of the sensor after addition of lactic acid was significantly reduced particularly in the case of a high concentration (40 mM) of lactic acid.

The reason why the current response value of this sensor decreased can be understood as that the pH of the measurement liquid deviated from the optimal pH of the enzyme when the lactic acid concentration was high as described above (see Table 1), and in addition, the pH of the reaction field decreased due to protons generated by the enzyme reaction, so that the enzyme was deactivated and the enzyme was not in a suitable active state.

On the other hand, as described above, in Example 1A (an aspect in which polyvinylimidazole is added to the sensor reagent liquid), since the current response value of the sensor to lactic acid at each concentration (10 mM to 40 mM) was stable as compared with Comparative Example 1A (an aspect in which polyvinylimidazole is not added to the sensor reagent liquid), it is considered that polyvinylimidazole played a role of pH buffering ability, and it was suitably suppressed that the pH in the vicinity of the enzyme was out of the optimal pH range of the enzyme.

That is, in Example 1A, it is considered that the deactivation of the lactate oxidase was suppressed by the pH buffering ability of polyvinyl imidazole.

### [Durability]

In Comparative Example 1A, it was found that the responsiveness of the sensor to each concentration (10 mM to 40 mM) of lactic acid decreased with the elapse of the storage period (before storage of the sensor ⇒ 7 days after storage ⇒ 13 days after storage).

On the other hand, in Example 1A, it was found that the responsiveness of the sensor to each concentration (10 mM to 40 mM) of lactic acid was maintained even after elapse of the storage period (before storage of the sensor ⇒ 7 days after storage ⇒ 13 days after storage).

From the above, it was found that the sensor durability can be improved by adding polyvinylimidazole to the sensor reagent liquid.

### Example 2A

### [Production of biosensor]

A biosensor was produced through the following steps (1) to (6).

### ● Step (1): Production of electrode

An electrode produced by sputtering platinum on an insulating substrate was immersed in a Nafion solution (produced by FUJIFILM Wako Pure Chemical Corporation), pulled up, and dried repeatedly a plurality of times to coat the platinum electrode surface with Nafion.

### ● Step (2): Preparation of reagent liquid

The following reagents were mixed so as to have the following final concentrations, and reacted for about 1 hour to prepare a reagent liquid.
· Lactate oxidase (LOX T-47: manufactured by Asahi Kasei Pharma Corporation) final concentration: 40U/mL
· Bovine serum albumin (manufactured by FUJIFILM Wako Pure Chemical Corporation), final concentration: 12.5 mg/mL
· Poly-L-histidine hydrochloride (manufactured by Sigma-Aldrich Co. LLC.), final concentration: 13.7 mg/mL
· 25% glutaraldehyde solution (manufactured by FUJIFILM Wako Pure Chemical Corporation), final concentration: 0.03125% (w/v)

### ● Step (3): Application of reagent liquid

1 µL of the reagent prepared in the step (2) was applied onto the electrode produced in the step (1), and dried over overnight. As a result, a reagent layer was formed on the working electrode.

### ● Step (4): Preparation of polymer solution for protective membrane

The following reagents were mixed so as to have the following final concentrations to prepare a polymer solution for a protective membrane.
· Poly(ter.Butyl methacrylate-b-4-vinylpyridine)(manufactured by Polymer Source, hereinafter referred to as "tBuMA4VP") final concentration 7.11% (w/v)
· Random copolymer of tripropylene glycol methyl ether methacrylate and styrene-4-vinylpyridine (manufactured by Nard, hereinafter referred to as "TGMAS4VP") at a final concentration of 0.89% (w/v)
· Poly(ethylene glycol) diglycidyl ether (manufactured by Sigma-Aldrich Co. LLC., hereinafter referred to as "PEGDGE") final concentration: 0.98% (w/v)
· HEPES buffer solution (pH8.0) final concentration 5 mM

tBuMA4VP, TGMAS4VP, and PEGDGE were dissolved in ethanol and used. The HEPES buffer solution was prepared by dissolving 2-[4-(2-hydroxyethyl)-1-piperazinyl] ethanesulfonic acid (manufactured by DOJINDO LABORATORIES) in Milli-Q water and adjusting the pH with sodium hydroxide.

### ● Step (5): Application of protective membrane solution

The operation of immersing the electrode produced in the step (3) in the polymer solution for a protective membrane, pulling up the electrode, and drying the electrode was repeated a plurality of times to form a protective membrane on each electrode. Thus, a protective membrane was formed on the reagent layer.

### ● Step (6): Formation of electrode part

The sensor electrode produced in the step (5) was used as a working electrode, and a palladium electrode as a counter electrode and an Ag/AgCl electrode as a reference electrode were combined to produce a three-electrode type electrode part.

### Comparative Example 2A

Comparative Example 2A is different from Example 2A in that poly-L-histidine hydrochloride is not added in the step (2): preparation of a reagent liquid. The other points are the same as those in Example 2A, and thus description thereof is omitted in order to avoid duplication of description.

### [Electrochemical measurement using biosensor]

Next, electrochemical measurement was performed using the biosensors produced in Comparative Example 2A and Example 2A.

For the electrochemical measurement using the biosensor, a current response value in an RPMI culture medium to which a 20 mM lactic acid solution was added in advance was continuously measured by an amperometric method using a potentiostat in a thermostatic bath at 37°C.

Specifically, the current response value of the sensor to lactic acid in the RPMI culture medium to which lactic acid was added in advance was continuously measured until 150 hours elapsed after the start of the measurement.

As an RPMI culture medium, RPMI-1640 Medium (R1383 manufactured by Sigma-Aldrich Co. LLC.) was used, and a solution prepared by adding L-lactic acid (manufactured by Tokyo Chemical Industry Co., Ltd.) at 20 mM, inactivated fetal bovine serum (manufactured by Thermo Fisher Scientific Co., Ltd.) at 10%, penicillin-streptomycin-amphotericin B suspension (manufactured by FUJIFILM Wako Pure Chemical Corporation) at 1%, and MES(2-Morpholinoethanesulfonic acid monohydrate) (manufactured by DOJINDO LABORATORIES) and MOPS (3-Morpholinopropanesulfonic acid) (manufactured by DOJINDO LABORIES) as buffer solution components at final concentrations of 25 mM and adjusting the pH to 7.4 and 6.5 was used.

### [Measurement results]

### ● Relation between measurement elapsed time and current response value in each of Example 2A and Comparative Example 2A

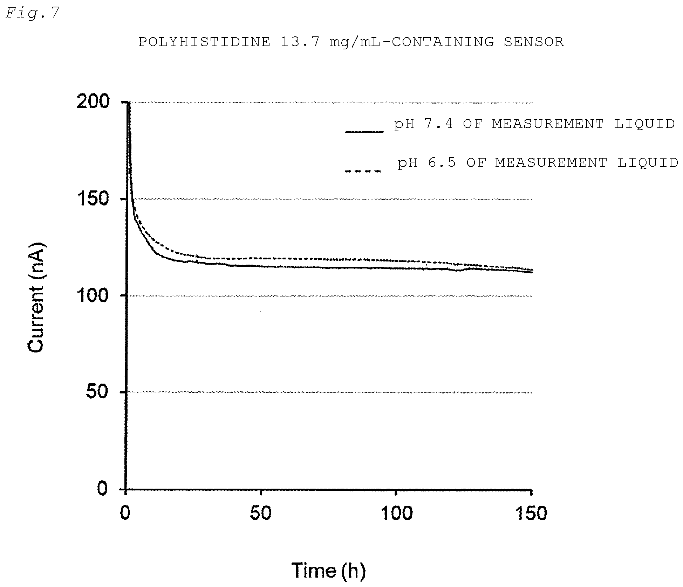

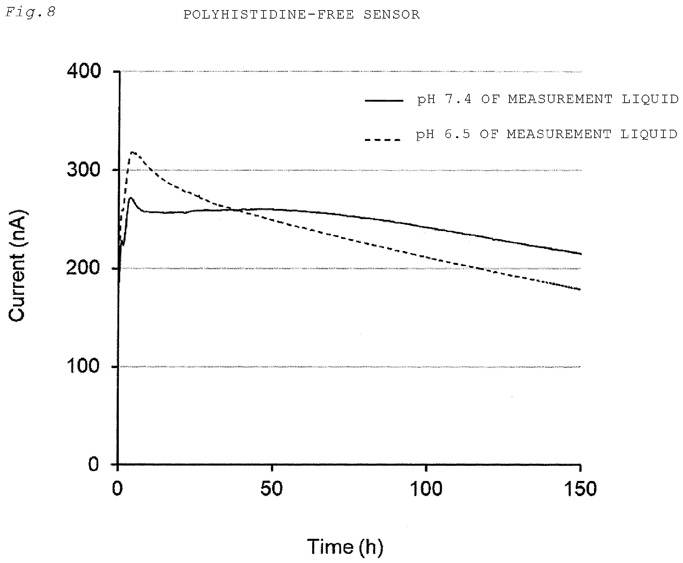

The relation between the measurement elapsed time and the current response value in Example 2A is shown in Fig. 7, and the relation between the measurement elapsed time and the current response value in Comparative Example 2A is shown in Fig. 8.

### [Sensor responsiveness]

As shown in Figs. 7 and 8, in Comparative Example 2A (an aspect in which poly-L-histidine is not added to the sensor reagent liquid), the current response value of the sensor was different depending on the pH of the measurement solution, and thus it was found that the responsiveness of the sensor to lactic acid was affected by the pH.

On the other hand, in Example 2A (an aspect in which poly-L-histidine is added to the sensor reagent liquid), it was found that the current response value of the sensor to lactic acid was substantially the same even when there was a difference in the pH of the measurement solution.

From these measurement results, it is considered that poly-L-histidine plays a role of pH buffering ability, and the pH dependence of the responsiveness of the lactic acid sensor is suppressed. That is, it is considered that in Example 2A, pH change in the vicinity of the reagent was suppressed by the pH buffering ability by poly-L-histidine, and the activity change of lactate oxidase due to pH decrease did not occur.

### [Durability]

In Comparative Example 2A, it was found that the current response value of the sensor decreased with the elapse of the measurement time. On the other hand, in Example 2A, it was found that the current response value of the sensor was maintained even after the measurement time elapsed.

From these measurement results, it was found that poly-L-histidine playing a role of the above-described pH buffering ability can also contribute to the maintenance of the activity of lactate oxidase.

From the above, it was found that by adding poly-L-histidine to the sensor reagent liquid, the pH dependence of the sensor current response value is suppressed and the durability is improved.

Summarizing the measurement results of Examples 1A and 2A and Comparative Examples 1A and 2A, it was found that polyvinylimidazole and poly-L-histidine added to the sensor reagent liquid commonly have a heterocyclic nitrogen group, that is, have a proton accepting group.

From the above, it has been found that when the reagent layer as a constituent of the finally obtained biosensor has a configuration containing a polymer containing a proton accepting group in a repeating unit in addition to an enzyme, a proton in an ionization state that can be generated when lactic acid (corresponding to an analyte) is oxidized by an enzymatic reaction in the reagent layer can be suitably accepted.

That is, it was found that the pH of the reaction field can be suppressed from deviating from the optimal pH of the enzyme, and the pH of the reaction field can be maintained within a certain range. Therefore, it has been found that deactivation of the enzyme can be avoided even when the analyte is continuously measured, pH dependence of the responsiveness of the biosensor can be suppressed, and durability can be improved.

Note that the present invention is not limited to the illustrated embodiments, and various improvements and design changes can be made without departing from the gist of the present invention.

An embodiment of the present invention (invention A) as described above includes the following preferred aspects.
<1A> A reagent layer including a polymer containing a proton accepting group in a repeating unit and an oxidoreductase that oxidizes or dehydrogenates an analyte.
<2A> The reagent layer according to <1A>, wherein the polymer has pH buffering ability.
<3A> The reagent layer according to <1A> or <2A>, wherein the polymer has a buffering ability in a pH range in which the oxidoreductase can maintain activity.
<4A> The reagent layer according to any one of <1A> to <3A>, wherein the proton accepting group is a heterocyclic nitrogen group.
<5A> The reagent layer according to any one of <1A> to <3A>, wherein the proton accepting group is at least one selected from the group consisting of a phosphate group, a sulfo group, and a carboxy group in an ionization state.
<6A> The reagent layer according to <4A>, wherein the heterocyclic nitrogen group is at least one selected from the group consisting of an imidazole group, a pyridyl group, an indolyl group, a quinolyl group, an isoquinolyl group, a tetrahydroquinolyl group, a thiazole group, an indolidyl group, an imidazopyridyl group, an acridinyl group, a tetrazole group, a triazole group, a pyrazyl group, a morpholyl group, and a piperazyl group.
<7A> The reagent layer according to any one of <1A> to <4A>, wherein the polymer is polyvinylimidazole.
<8A> The reagent layer according to any one of <1A> to <4A>, wherein the polymer is poly-L-histidine.
<9A> The reagent layer according to any one of <1A> to <3A> and <5A>, wherein the polymer is sodium polyphosphate.
<10A> The reagent layer according to any one of <1A> to <9A>, wherein the polymer has a weight average molecular weight of 10,000 or more.
<11A> A biosensor including the reagent layer according to any one of <1A> to <10A>.
<12A> The biosensor of <11A>, wherein the biosensor is a lactic acid sensor.

### INDUSTRIAL APPLICABILITY

The biosensor including the reagent layer according to one embodiment of the present invention can be used for suitable measurement of an analyte.

### REFERENCE SIGNS LIST

10 Reagent layer
20 Protective membrane
30 Working electrode
100 Biosensor
A Polymer
B Enzyme

Hereinafter, an embodiment of the present invention (invention B) is described.

### TITLE OF THE INVENTION: POLYMER, REAGENT COMPRISING POLYMER, BIOSENSOR COMPRISING REAGENT LAYER COMPRISING POLYMER, AND METHOD FOR SYNTHESIZING POLYMER

### TECHNICAL FIELD

The present invention relates to a polymer, a reagent containing a polymer, a biosensor including a reagent layer including a polymer, and a method for synthesizing a polymer.

### BACKGROUND ART

In recent years, in various fields such as a medical field, a biosensor can be used to measure an analyte (substance to be detected) in a cell or the like. As a method for measuring an analyte, for example, an electrochemical measurement method can be used. A biosensor in an electrochemical measurement method includes a reagent layer containing an enzyme provided on an electrode (corresponding to a working electrode) and a protective membrane covering the reagent layer. Various analytes can be measured by appropriately selecting an enzyme that acts as a substrate with an analyte to be measured. For example, by selecting lactate oxidase (LO_{X}) as an enzyme, the concentration of lactic acid as an analyte can be measured.

### PRIOR ART DOCUMENT

### PATENT DOCUMENT

Patent Document 2: JP-A-2010-517054

### SUMMARY OF THE INVENTION

### PROBLEMS TO BE SOLVED BY THE INVENTION

Here, the present inventors have newly found that there is a matter to be improved for the conventional biosensor. Specifically, protons (H⁺) are released when the analyte in the cell chemically reacts with the enzyme, and the pH of the reagent layer of the biosensor may decrease accordingly. As a method for suppressing this pH decrease, a constitution is considered in which the reagent layer further contains a phosphate buffering agent in addition to the enzyme.

However, since the molecular weight of the buffering agent is relatively small, it may be difficult for the buffering agent to remain in the reagent layer. Therefore, it is difficult to secure a predetermined amount of buffering agent in the reagent layer, and there is a possibility that it becomes difficult to suppress a pH decrease. In a biosensor, a pH decrease can lead to deterioration of an enzyme contained in a reagent layer, and therefore there is a possibility that suitable measurement of an analyte becomes difficult. From the above, a polymer capable of suppressing pH decrease and providing water solubility during reagent formation is desired.

Therefore, an object of the present invention is to provide a polymer capable of suppressing pH decrease and providing water solubility, a reagent containing a polymer, a biosensor including a reagent layer containing a polymer, and a method for synthesizing a polymer.

### SOLUTIONS TO THE PROBLEMS

To achieve the above object, in an embodiment of the present invention,
a polymer including a proton accepting group and an ionic group as a repeating unit is provided.

In an embodiment of the present invention, a reagent containing an oxidoreductase that oxidizes or dehydrogenates the polymer and the analyte is provided.

In one embodiment of the present invention, there is provided a biosensor including a reagent layer containing the reagent.

To achieve the above object, in an embodiment of the present invention,
a method for synthesizing a polymer including
a first step of preparing a first solution containing a proton accepting group;
a second step of preparing a second solution containing an ionic group;
a third step of adding the second solution to the first solution and mixing the first solution with the second solution; and
a fourth step of performing a polymerization reaction using a mixture containing the first solution and the second solution in the presence of a polymerization initiator
is provided.

### EFFECTS OF THE INVENTION

According to an embodiment of the present invention, it is possible to provide a biosensor including a polymer capable of suppressing pH decrease and providing water solubility, a reagent containing the polymer, and a reagent layer containing the polymer.

### BRIEF DESCRIPTION OF THE DRAWINGS

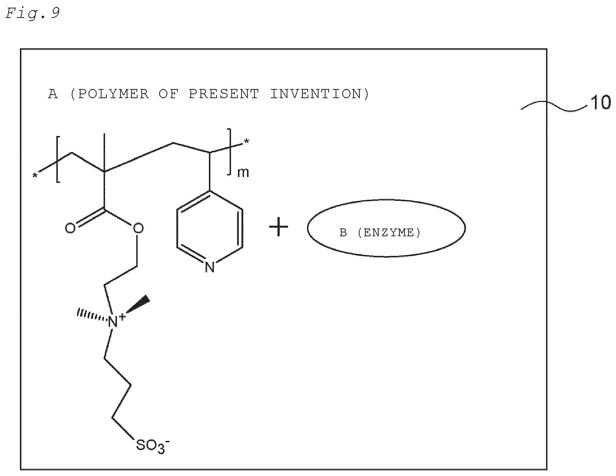

[Fig. 9] Fig. 9 is a cross-sectional view schematically showing a configuration of a reagent layer containing a reagent according to an embodiment of the present invention.

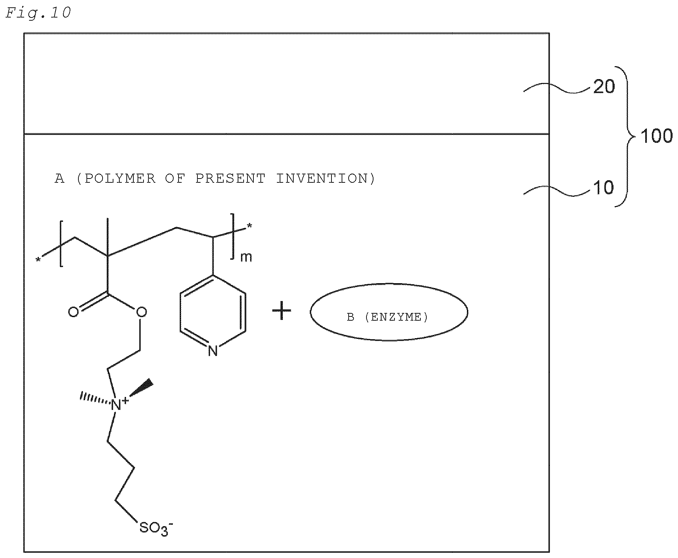

[Fig. 10] Fig. 10 is a cross-sectional view schematically illustrating a main configuration of a biosensor 100 according to an embodiment of the present invention.

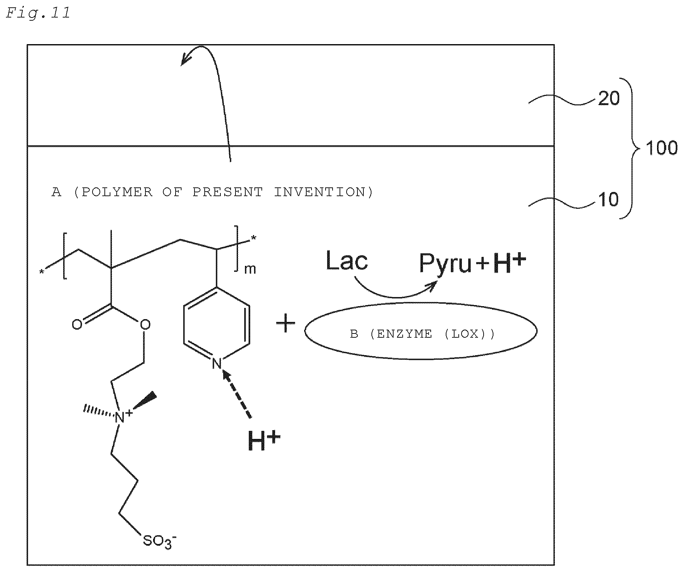

[Fig. 11] Fig. 11 is a cross-sectional view schematically illustrating an action mechanism provided based on a main configuration of a biosensor 100 according to an embodiment of the present invention.

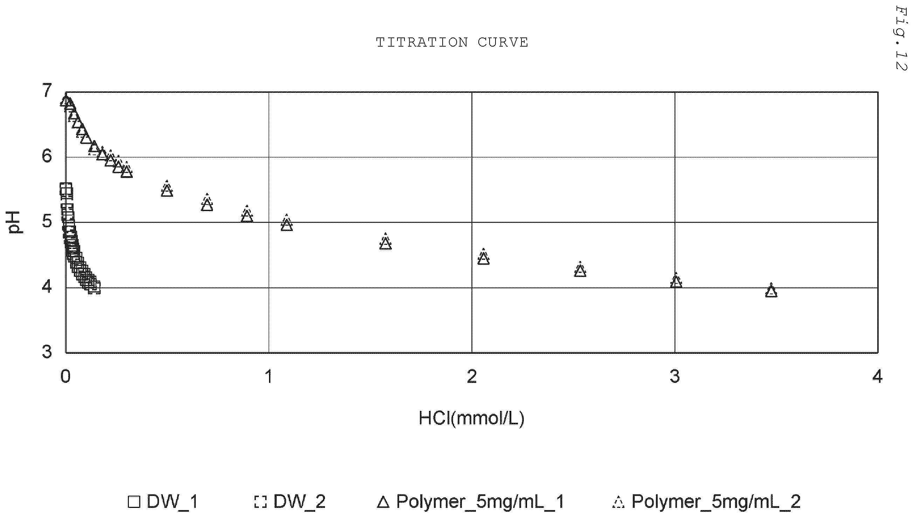

[Fig. 12] Fig. 12 is a graph showing a relation between HCl added dropwise and solution pH in Example 2B and Comparative Example 2B.

### DETAILED DESCRIPTION

Hereinafter, an embodiment of the present invention (invention B) is specifically described.

### [Polymer of the present invention]

First, a polymer according to an embodiment of the present invention is described.

The present inventors have intensively studied the constitution of a new polymer capable of suppressing pH decrease and providing water solubility. As a result, the present inventors have newly found that when a repeating unit of a polymer has the following two characteristic groups in combination, it is possible to suppress the pH decrease and to provide water solubility.

Specifically, the polymer according to an embodiment of the present invention is characterized by including a "proton accepting group" and an "ionic group" as the repeating unit. In the present specification, the "proton accepting group" refers to a functional group capable of accepting a proton (H⁺) contained in a repeating unit of a polymer. In the present specification, the "ionic group" refers to an ionic functional group that is contained in a repeating unit of a polymer and is capable of providing hydrophilicity.

In an embodiment of the present invention, the polymer has the above characteristics, and thus a proton (H⁺) can be received by the presence of a proton accepting group in the repeating unit. In addition, the presence of an ionic group in the repeating unit makes it possible to suitably provide hydrophilicity, thereby making it possible to provide water solubility. Since the polymer is composed of a large number of repeating units, it is possible to effectively provide the proton accepting function and the water-soluble donating function of the repeating units.

As described later, in the case of using a biosensor including a reagent layer containing the polymer and the enzyme, an effective proton accepting function by the polymer makes it possible to suitably accept protons (H⁺) that can be released when an analyte (for example, lactic acid) in a cell or the like chemically reacts with the enzyme. This makes it possible to suppress a pH decrease in the reagent layer and to suppress deterioration of the enzyme contained in the reagent layer. As a result, the analyte can be suitably measured. In addition, since the polymer can be suitably dissolved in the solution containing the enzyme due to the effective water-soluble donating function by the polymer, the reagent layer can be suitably formed as a whole.

In one embodiment of the present invention, from the viewpoint of effective proton acceptance and water-soluble donation, the weight average molecular weight (Mw) of the polymer can be 10,000 or more, 20,000 or more, 30,000 or more, 40,000 or more, 50,000 or more, 60,000 or more, 70,000 or more, 80,000 or more, 90,000 or more, 100,000 or more, 110,000 or more, preferably 90,000 or more, and for example, 98,000. The upper limit of the weight average molecular weight (Mw) of the polymer may be 1 million or less from the viewpoint of water solubility and viscosity.

In a preferred aspect, the ionic groups described above may be zwitterionic type. The term "zwitterionic type" as used herein refers to a type in which the repeating unit of the polymer of the present invention has both a positively charged (+) ionic group and a negatively charged (-) ionic group. When the ionic group is a zwitterionic type, the number of ionic groups contained in the repeating unit is at least doubled, and therefore hydrophilicity can be more suitably provided. This makes it easier to provide water solubility. For example, a zwitterionic type ionic group can include quaternary ammonium groups in the cationic state and a sulfo group or a carboxy group in the anionic state.

In a preferred aspect, the proton accepting group described above may be a heterocyclic nitrogen group containing an atom having an unshared electron pair. In the present specification, the "heterocyclic nitrogen group" means a group having a ring structure containing nitrogen. Since nitrogen has high electronegativity and high electron-withdrawing property, it is in a state of easily accepting protons. In addition, from the viewpoint of suitably providing a buffering function by proton acceptance, the pKa of the heterocyclic nitrogen group may be 4 to 8 in consideration of the pH range of the reagent layer containing the polymer of the present invention.

In one example, the heterocyclic nitrogen group can be a pyridyl group.

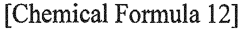

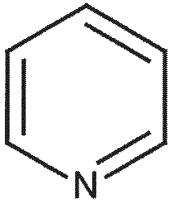

In another example, the heterocyclic nitrogen group can be an imidazole group.

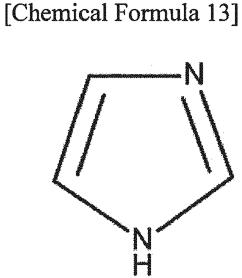

In another example, the heterocyclic nitrogen group can be a benzimidazole group.

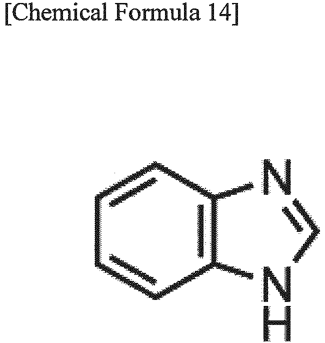

In another example, the heterocyclic nitrogen group can be an isoquinolyl group.

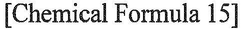

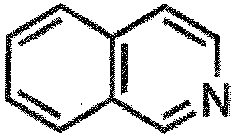

In one embodiment, the heterocyclic nitrogen group may be at least one selected from the group consisting of a pyridyl group, an imidazole group, a benzimidazole group, and an isoquinolyl group. That is, the heterocyclic nitrogen group may be composed of two or more selected from them.

As a specific example, the polymer according to an embodiment of the present invention may have the following structure.

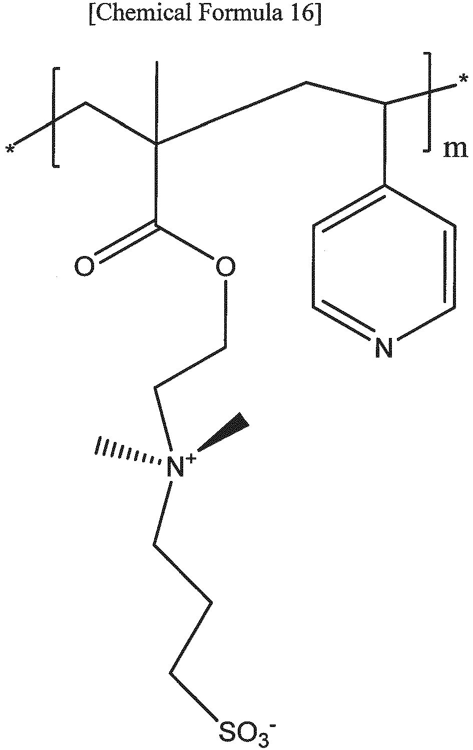

In the repeating unit of the polymer, the first moiety including an ionic group may comprise 3-[[2-(methacryloyloxy)ethyl]dimethylammonio]propane-1-sulfonate. In the repeating unit of the polymer, the second moiety having a proton accepting group may contain 4-vinylpyridine. That is, the repeating unit of the polymer may include a moiety derived from 4-vinylpyridine and a moiety derived from 3-[[2-(methacryloyloxy)ethyl]dimethylammonio]propane-1-sulfonate. The moiety derived from 4-vinylpyridine and the moiety derived from 3-[[2-(methacryloyloxy)ethyl]dimethylammonio]propane-1-sulfonate are continuous in a state of being covalently bonded to each other as shown in Chemical Formula 5 above.

### [Method for synthesizing polymer of present invention]

Hereinafter, a method for synthesizing a polymer according to an embodiment of the present invention is described.

A method for synthesizing a polymer according to an embodiment of the present invention includes:
a first step of preparing a first solution containing a proton accepting group;
a second step of preparing a second solution containing an ionic group;
a third step of adding the second solution to the first solution and mixing the first solution with the second solution; and
a fourth step of performing a polymerization reaction using a mixture containing the first solution and the second solution in the presence of a polymerization initiator.

### (First step)

In the first step, a first solution containing a proton accepting group is prepared as described above. As an example, a first solution containing a heterocyclic nitrogen group as the proton accepting group is prepared. In addition, from the viewpoint of suitably providing a buffering function by proton acceptance, the pKa of the heterocyclic nitrogen group may be 4 to 8 in consideration of the pH range of a reagent layer to be formed later. As an example, the heterocyclic nitrogen group may be at least one selected from the group consisting of a pyridyl group, an imidazole group, a benzimidazole group, and an isoquinolyl group. For example, the first solution can be obtained by mixing the following 4-vinylpyridine (which may be referred to as 4VP) containing a proton accepting group with an aprotic polar solvent (for example, dimethyl sulfoxide (DMSO)).

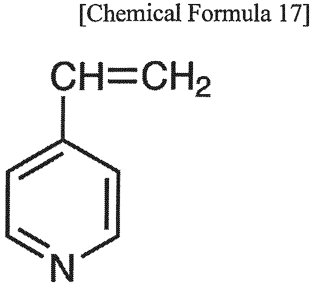

### (Second step)

In the second step, a second solution containing an ionic group is prepared as described above. This substance containing an ionic group is dissolved in water as a solvent for several seconds to prepare a second solution. The ionic group contained in the second solution is preferably a zwitterionic type from the viewpoint of suitably providing water solubility. As the zwitterionic type ionic group, those containing a quaternary ammonium group in a cationic state and a sulfo group or a carboxy group in an anionic state can be selected.

As an example, as a substance containing an ionic group, the following 3-[[2-(methacryloyloxy)ethyl]dimethylammonio]propane-1-sulfonate (which may be referred to as MAS) is added to water as a solvent, and is dissolved in an ultrasonic cleaner for 3 seconds or more and 7 seconds or less, for example, for 5 seconds. Thus, the second solution is prepared.

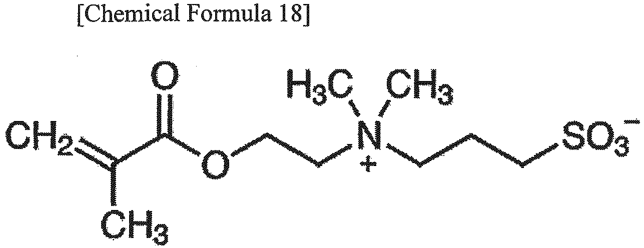

### (Third step)

In the third step, the second solution is added to the first solution and mixed. Specifically, in the third step, the second solution prepared in the second step is immediately added to the first solution prepared in the first step, for example, within 0.5 seconds or more and 4 seconds, and uniform mixing is performed.

### (Fourth step)

In the fourth step, a polymerization reaction is performed using a mixture containing the first solution and the second solution in the presence of a polymerization initiator. As the polymerization initiator, for example, the following powdery azobisisobutyronitrile (which may also be referred to as AIBN) can be used as an initiator of a radical reaction.

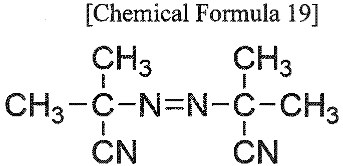

Specifically, in a three-necked flask, an initiator is added to the mixture, and then the inside of the flask is replaced in a nitrogen environment, and then a polymerization reaction is started. After starting the polymerization reaction at a predetermined reaction temperature and stirring rate, the polymerization reaction is terminated after a predetermined reaction time elapses. After completion of the polymerization reaction, Milli-Q water is added to dissolve the polymerization reaction product. Thereafter, for purification of the polymerization reaction product, the dissolved liquid is dropped into hexane to form a precipitate. Thereafter, the precipitate is collected, evaporated, and finally dried under reduced pressure. As described above, the polymer according to one embodiment of the present invention can be obtained.

### [Reagent containing polymer of present invention]

Hereinafter, a reagent containing a polymer according to an embodiment of the present invention is described. Fig. 9 is a cross-sectional view schematically showing a configuration of a reagent layer containing a reagent according to an embodiment of the present invention.

The polymer according to an embodiment of the present invention can be used in a reagent containing an enzyme. That is, in one embodiment of the present invention, the reagent may contain at least the polymer A and the enzyme B. The reagent layer 10 is a layer containing this reagent (see Fig. 9). The enzyme B may be an oxidoreductase that oxidizes or dehydrogenates an analyte. The term "oxidoreductase" as used herein means a biochemical substance capable of specifically catalyzing oxidation or reduction of an analyte.

Examples of the oxidoreductase include glucose oxidase, lactate oxidase, cholesterol oxidase, bilirubin oxidase, glucose dehydrogenase, lactate dehydrogenase, amino acid oxidase, amino acid dehydrogenase, glutamate oxidase, glutamate dehydrogenase, fructosylamino acid oxidase, fructosyl peptide oxidase, 3-hydroxybutyrate dehydrogenase, alcohol oxidase, and/or alcohol dehydrogenase.

The oxidoreductase described above can be used for detection of amino acids such as glucose, lactic acid, cholesterol, bilirubin, glutamine, and glutamic acid, glycosylated amino acids, or glycosylated peptides, ketone bodies (3-hydroxybutyric acid), alcohols, and the like. The amount of the oxidoreductase is, for example, 0.01U to 100U (µmol/min), preferably 0.05U to 10U, and more preferably 0.1U to 5U per biosensor described later or per measurement.

The reagent may further contain a mediator and/or conductive particles in addition to the polymer and the enzyme. Examples of the conductive fine particles include carbon black and carbon nanotubes.

The term "mediator" as used herein means, in a broad sense, an oxidation-reduction substance that mediates electron transfer, and in a narrow sense, a substance that is responsible for transfer of electrons generated by an oxidation-reduction reaction of an analyte in the following biosensor.

Although not particularly limited, examples of the mediator include a metal complex (for example, an osmium complex, a ruthenium complex, an iron complex, or the like), a quinone compound (examples thereof include benzoquinone, naphthoquinone, phenanthrenequinone, phenanthroline quinone, anthraquinone, and derivatives thereof), a phenazine compound, a viologen compound, a phenothiazine compound, and a phenol compound.

More specifically, As the mediator, one or more selected from the group consisting of phenazine derivatives such as potassium ferricyanide, hexaammine ruthenium, ferrocene, poly(1-vinylimidazole)-bis(bipyridine)chloroosmium, hydroquinone, 2-methyl-1,4-benzoquinone, 1,2-naphthoquinone-4-sulfonate, 9,10-phenanthrenequinone-2-sulfonate, 9,10-phenanthrenequinone-2,7-disulfonate, 1,10-phenanthroline-5,6-dione, anthraquinone-2-sulfonate, 1-methoxy-5-methylphenazinium methylsulfate and 1-methoxy-5-ethylphenazinium ethylsulfate, methylviologen, benzylviologen, methylene blue, methylene green, 2-aminophenol, 2-amino-4-methylphenol, and, 2,4-diaminophenol are used.

Examples of the salt include, and are not limited to, a sodium salt, a potassium salt, a calcium salt, a magnesium salt, and a lithium salt. The blending amount of the mediator is not particularly limited, and is, for example, 0.1 pmol to 1000 µmol, preferably 10 pmol to 500 µmol, and more preferably 500 pmol to 100 µmol, per measurement or per biosensor described below.

### [Biosensor]

Hereinafter, a biosensor according to an embodiment of the present invention is described. Fig. 10 is a cross-sectional view schematically illustrating a main configuration of the biosensor 100 according to the embodiment of the present invention.

The "biosensor" as used herein is a measurement device that converts a chemical change generated by a molecular recognition reaction between a substrate (corresponding to an analyte) and an enzyme (corresponding to a receptor) into an electric signal using a combination of an enzyme-substrate and the like, and measures the metabolic rate, concentration, and the like of the analyte according to the intensity of the obtained electric signal.

A biosensor 100 according to an embodiment of the present invention includes a reagent layer 10 and a protective membrane 20 disposed on an electrode part formed on a substrate surface (see Fig. 10). In the present embodiment, for the sake of convenience, it should be noted that the reagent layer 10 and the protective membrane 20 of the biosensor 100 are illustrated, and illustration of other electrode parts and the like is omitted.

As the substrate, an insulating substrate can be used. Although not particularly limited, the substrate can be made of a material such as polyethylene terephthalate, polyamide, or polyimide having a thickness of several hundred µm. In one embodiment, the electrode part of biosensor 100 can be inserted into the liquid culture medium (liquid sample) provided in the culture tank. After the biosensor 100 is inserted, a voltage can be applied to the electrode part.

The electrode part includes a working electrode and a counter electrode and/or a reference electrode. A reagent layer 10 containing a reagent containing the polymer and the enzyme of the present invention can be disposed on the surface of the working electrode.

The protective membrane 20 can be configured to cover the reagent layer 10 and the electrode part. The protective membrane 20 is configured to be able to permeate a specific component (lactic acid or the like) of the cells in the culture medium toward the working electrode while controlling the permeation rate of the specific component. In addition, the protective membrane 20 is configured to be able to suppress the components (the polymer, the enzyme, and the like of the present invention) contained in the reagent layer 10 on the working electrode from flowing out to the outside of the protective membrane 20. That is, the "protective membrane" used in the present specification is a membrane that contributes to suppression of leakage of a substance contained in the reagent layer to the outside of the protective membrane, and is a membrane having a hole through which an analyte present outside the protective membrane is movable to the reagent layer side via the protective membrane.

The protective membrane may be a biocompatible polymer membrane. The polymer constituting the protective membrane may be t-butyl acrylate having a heterocyclic nitrogen group. As the heterocyclic nitrogen group, for example, a pyridyl group, an imidazole group, or the like can be selected. Furthermore, the polymer constituting the protective membrane may further include Nafion having a fluorocarbon as a main chain and containing a sulfo group and a carboxyl group in a side chain.

Although not particularly limited, examples of the culture medium include RPMI-1640 culture medium, D-MEM culture medium, F12, and MEM culture medium. In the present invention, the culture medium does not contain an enzyme.

Under the above configuration, the analyte that moves from the culture medium to the reagent layer 10 via the protective membrane 20 in a state where the electrode part is immersed in the culture medium is oxidized in the reagent layer 10 using the enzyme as a catalyst and dissolved oxygen. The concentration of the analyte can be measured by electrically detecting hydrogen peroxide generated at that time.

Fig. 11 is a cross-sectional view schematically illustrating an action mechanism provided based on a main configuration of a biosensor 100 according to an embodiment of the present invention. Taking the case of detecting lactic acid as an example, lactic acid (corresponding to Lac in Fig. 11) that has moved from the culture medium to the reagent layer 10 through the protective membrane 20 is oxidized by an enzymatic reaction with an enzyme (corresponding to LOx in Fig. 11) in the reagent layer 10, and pyruvic acid (corresponding to Pyru in Fig. 11), hydrogen peroxide, and a proton in an ionization state (H+) can be formed. The metabolic rate of lactic acid can be measured by electrically measuring hydrogen peroxide generated during the formation. In this case, the biosensor 100 can function as a lactic acid sensor.

As described above, according to the polymer of the present invention, it is possible to effectively provide the proton accepting function and the water-soluble donating function. The biosensor 100 includes a reagent layer 10 containing the polymer A and the enzyme B. Therefore, due to the effective proton accepting function by the polymer A, the analyte (lactic acid in Fig. 11) that has moved into the reagent layer 10 can suitably accept a proton (H⁺) that can be released when the analyte chemically reacts with the enzyme B. This makes it possible to suppress a pH decrease in the reagent layer 10 and to suppress deterioration of the enzyme B contained in the reagent layer 10. As a result, the biosensor 100 can be directly inserted into the culture tank provided with the culture medium, and the analyte can be suitably measured.

In addition, since the polymer A can be suitably dissolved in the solution containing the enzyme B due to the effective water-soluble donating function by the polymer A, the reagent layer 10 can be suitably formed as a whole. Furthermore, as compared with a conventional buffering agent having a low molecular weight for suppressing pH decrease, the polymer A of the present invention has a high molecular weight, and thus tends to remain in the reagent layer 10, and can suitably suppress outflow to the culture medium side located outside the protective membrane 20.

In the conventional biosensor, there is an aspect in which the protective membrane mentioned above contains a heterocyclic nitrogen-containing polymer. This protective membrane merely contributes to limiting diffusion of the analyte into the working electrode which is a constituent of the sensor. On the other hand, in biosensor 100 of the present invention, reagent layer 10 (not protective membrane 20) which is a constituent contains a polymer, and the polymer has groups having different characteristics between a proton accepting group contributing to proton acceptance and an ionic group contributing to provision of water solubility. From the above, as compared with the aspect in which the protective membrane contains a heterocyclic nitrogen-containing polymer, the polymer of the present invention is different from the polymer of the present invention in both the provision site and the function (both functions of proton acceptability and water solubility).

### [Method for producing biosensor]

Hereinafter, an example of a method for producing the biosensor is described.

First, an insulating substrate is prepared. After the preparation of the insulating substrate, a conductive thin membrane (corresponding to a working electrode) is formed by depositing an insulating substrate selected from metals such as silver, platinum, and palladium by a sputtering method, a vapor deposition method, ion plating, or the like. The thickness of the conductive thin membrane may be 10 nm to several hundred nm. The counter electrode and/or the reference electrode may be disposed on the peripheral edge of the working electrode, for example, on the back side of the substrate. An insulating resist layer may be formed on parts other than these electrode forming parts.

Thereafter, a reagent having at least the polymer and the enzyme solution of the present invention is applied onto the working electrode. In addition to the polymer and the enzyme, the reagent may further contain a suspension of conductive particles such as carbon particles for imparting conductivity and an aqueous solution of a mediator. The reagent layer 10 can be suitably formed by drying the reagent applied to the working electrode. Finally, a protective membrane 20 is formed so as to cover the reagent layer 10 and the electrode part. As described above, biosensor 100 can be produced.

### Examples

Hereinafter, examples of the present invention (invention B) are described.

### Example 1B

### [Synthesis of polymer of present invention]

### (Implementation of first step)

As a first step, 4-vinylpyridine (which may be referred to as 4VP) and styrene were mixed with dimethyl sulfoxide (DMSO) to prepare a first solution.

The use amount, concentration, and product number are as follows.
[Use amount]
   · DMSO: 24.8 mL
   · 4VP: 3.539 mL
   · Styrene: 0.077 mL
[Concentration of 4VP]
   12.45 v/v%
[Concentration of styrene]
   0.27 v/v%
[Product number]
   · DMSO: 045-28335 (manufactured by FUJIFILM Wako Pure Chemical Corporation)
   · 4VP: V0150 (manufactured by Tokyo Chemical Industry Co., Ltd.)

### (Implementation of second step)

As a second step, 3-[[2-(methacryloyloxy)ethyl]dimethylammonio]propane-1-sulfonate (which may be referred to as MAS) was additionally dissolved in water as a solvent for 5 seconds with an ultrasonic cleaner. Thus, a second solution was prepared.

The use amount, concentration, and product number are as follows.
[Use amount]
   MAS: 9.36 g
   Milli-Q water: 5.0 mL
   [MAS concentration]
   65.2 (w/w%)
[Product number]
   MAS: M1971 (manufactured by Tokyo Chemical Industry Co., Ltd.)

### (Implementation of third step)

In the third step, the second solution was added to the first solution and mixed. Specifically, the second solution prepared in the second step was added to the first solution prepared in the first step within 1 second, and uniform mixing was performed. In such mixing, the mixing ratio (molar ratio) was styrene: 4VP : MAS = 1 : 49 : 50.

### (Fourth step)

In the fourth step, a polymerization reaction was performed using a mixture containing the first solution and the second solution in the presence of a polymerization initiator. Specifically, in a three-necked flask, an initiator (azobisisobutyronitrile (which may also be referred to as AIBN)) was added to the mixture. The solution temperature when AIBN was added was 24°C. Thereafter, the inside of the flask was replaced under a nitrogen environment, and a polymerization reaction was started.

The use amount, concentration, and product number of the initiator used are as follows.
[Use amount]
   AIBN: 0.064 g
[Concentration of AIBN]
   Powdered AIBN was added to a three-necked flask.
   AIBN concentration: 100%
[Product number]
   AIBN: 019-04932 (manufactured by FUJIFILM Wako Pure Chemical Corporation)

Thereafter, the polymerization reaction was started at a reaction temperature of 80°C and a stirring speed of 500rpm, and the polymerization reaction was terminated after an elapse of a reaction time of 42.5 hours. After completion of the polymerization reaction, Milli-Q water was added to dissolve the polymerization reaction product. Thereafter, the dissolved liquid was dropped into hexane to form a precipitate, and the precipitate was collected, evaporated, and finally dried under reduced pressure. As described above, the polymer of the present invention could be synthesized.

### Example 2B

### [Evaluation of characteristics of polymer of present invention]

The properties of the polymer of the present invention synthesized above were evaluated by the following method.

First, 1 mL of the polymer of the present invention (250 mg/mL) was additionally dissolved in 50 mL of DW (distilled water). To the resulting solution HCl at a concentration (0.1 mol/L) was added sequentially. The same procedure was performed again to ensure reproducibility. Thereafter, the pH was evaluated using a pH meter.

As Comparative Example comparable to Example 2B, HCl having a concentration (0.1 mol/L) was sequentially added to 50 mL of DW (distilled water). The same procedure was performed again to ensure reproducibility. Thereafter, the pH was evaluated using a pH meter.

The evaluation result 1 is shown in Fig. 12.

As shown in Fig. 12, in the comparative example in which HCl having a concentration (0.1 mol/L) was sequentially added to the DW without using the polymer of the present invention, the pH immediately after addition of HCl was started from a state in which the pH at the start of addition of HCl was around 5.5, and the pH immediately decreased to around 4. Specifically, the pH was around 4 when the concentration of added HCl was around 0.14 mmol/L.

On the other hand, in Example 2B in which HCl having a concentration (0.1 mol/L) was sequentially added to a solution in which the polymer of the present invention was added to DW, the pH finally reached approximately 4 when the concentration of added HCl reached approximately 3.4 mmol/L from a state in which the pH at the start of addition of HCl was near 7. In Example 2B, 1 mL of the polymer of the present invention (250 mg/mL) was suitably added to 50 mL of DW (distilled water). From the above, it was found that the polymer of the present invention has a function of suppressing pH decrease and a water-soluble donating function.

The weight average molecular weight (Mw) and the number average molecular weight (Mn) of the obtained polymer of the present invention were measured using GPC (Gel Permeation Chromatography: Gel Permeation Chromatography). The measurement results of each average molecular weight were as follows.
· Weight average molecular weight (Mw): 98000
· Number average molecular weight (Mn): 13000

### Example 3B

[1. Synthesis of polymer], [2. Production of sensor containing synthesized polymer], and [3. Immersion experiment of sensor] were performed through the following steps. Note that, from the viewpoint of avoiding overlapping with the contents described in Example 1B, the contents of the overlapping part are omitted.

### [1. Synthesis of polymer]

### (Implementation of first step)

As a first step, 4-vinylpyridine and styrene were mixed with dimethyl sulfoxide (DMSO) to prepare a first solution. The first solution preparation conditions in the first step are the same as those in Example 1B.

The use amount, concentration, and product number are as follows.
[Use amount]
   · DMSO: 24.8 mL
   · 4VP: 3.539 mL (105.14/mol)
   · Styrene: 0.077 mL (104.15/mol)
[Concentration of 4VP]
   12.45 v/v%
[Concentration of styrene]
   0.27 v/v%
[Product number]
   · DMSO: 045-28335 (manufactured by FUJIFILM Wako Pure Chemical Corporation)
   · 4VP: V0150 (manufactured by Tokyo Chemical Industry Co., Ltd.)

### (Implementation of second step)

As a second step, 3-[[2-(methacryloyloxy)ethyl]dimethylammonio]propane-1-sulfonate was added to water as a solvent to prepare a second solution. The second solution preparation conditions in the second step are the same as those in Example 1B.

The use amount, concentration, and product number are as follows.
[Use amount]
   MAS: 9.36 g (279.35/mol)
   Milli-Q water: 5.0 mL
[MAS concentration]
   65.2 (w/w%)
[Product number]
   MAS: M1971 (manufactured by Tokyo Chemical Industry Co., Ltd.)

### (Implementation of third step)

In the third step, the second solution was added to the first solution and mixed. The mixing ratio (molar ratio) was styrene : 4VP : MAS = 1 : 49:50. The addition and mixing conditions of the first solution and the second solution in the third step are the same as those in Example 1B.

### (Fourth step)

In the fourth step, a polymerization reaction was performed using a mixture containing the first solution and the second solution in the presence of a polymerization initiator. The polymerization reaction conditions in this fourth step are the same as those in Example 1B.

After completion of the polymerization reaction, Milli-Q water was added to dissolve the polymerization reaction product. Thereafter, the dissolved liquid was dropped into hexane to form a precipitate, and the precipitate was collected, evaporated, and finally dried under reduced pressure. As described above, the polymer of the present invention could be synthesized. In the obtained polymer, the molecular weight of the polymer basic component unit composed of 4VP and MAS was 191.63.

After the polymer synthesis, 250.0 mg of the polymer was weighed, and 1 mL of DW (distilled water) was added to prepare a polymer solution. Thus, the polymer solution concentration was 250 mg/mL(0.25 mg/µL, 1304.6 mmol/L).

### [2. Production of sensor containing synthesized polymer]

After the synthesis of the polymer, a sensor was produced through the following steps.

### ● (1) Production of working electrode

A conductive thin membrane was formed by printing carbon ink XC-3180 (manufactured by Fujikura Kasei Co., Ltd.) on an insulating substrate using a screen printer (manufactured by Celia Corporation). As the insulating substrate, PET W400J (manufactured by Mitsubishi Corporation) was used.

### ● (2) Production of water-repellent resist layer

A resist ink XB-3342 (manufactured by Fujikura Kasei Co., Ltd.) was printed on the working electrode using a screen printer (manufactured by Celia Corporation) to form a water-repellent resist.

### ● (3) Corona discharge treatment

The working electrode was subjected to a surface treatment using a corona discharge surface modification apparatus (manufactured by Shinko Electric Instrument Co., Ltd.).

### ● (4) Production of water-repellent film layer

A fluorine-based water-repellent film SS4A (produced by NiPPA Co., Ltd.) was attached onto the water-repellent resist layer to produce a water-repellent film layer.

### ● (5) Application of polymer solution

Onto the working electrode produced in the step (1), 1 µL of the 250 mg/mL polymer solution formed in [1. Synthesis of polymer] was applied and dried at room temperature to form a polymer membrane.

### ● (6) Preparation of solution for protective membrane and application of solution for protective membrane

After the drying, 0.6 mg of a 16.125 wt% neutralized Nafion solution for coating was applied onto the polymer membrane, and dried at room temperature to form a Nafion membrane. Subsequently, after the drying, 0.6 mg of a 4VP-tBuMA solution for coating was applied onto the Nafion membrane, and dried at room temperature to form a 4VP-tBuMA membrane. Thus, a protective membrane was formed on the polymer membrane.

### (Preparation of neutralized Nafion solution for coating)

The neutralized Nafion solution for coating was prepared through the following steps. Specifically, 26250.7 mg of 20 wt%(product number 663492, manufactured by Sigma-Aldrich Co. LLC) of the Nafion dispersion liquid was added to the bottle. Then, 7355.2 mg of ethanol (product number 057-00451, manufactured by FUJIFILM Wako Pure Chemical Corporation) was added into the bottle. Then, 1394.08 mg of a 5 mol/L aqueous sodium hydroxide solution (product number: 196-05375, manufactured by FUJIFILM Wako Pure Chemical Corporation) was added to the bottle. Finally, stirring with VORTEX-GENIE 2 for 60 seconds and sonication (sonication) for 5 seconds were repeated to completely dissolve the precipitate. Thus, a neutralized Nafion solution for coating was prepared.

### (Preparation of 4VP-tBuMA solution for coating)

The 4VP-tBuMA solution for coating was prepared through the following steps. Specifically, prior to the preparation of the 4VP-tBuMA solution for coating, 4.0 g of 4VP-tBuMA(product number P42487, [molecular weight ratio] 4VP : tBuMA = 120,000 : 270,000, manufactured by Polymer source Co., Ltd.) was added to a bottle, then 36 g of ethanol (Product No. 057-00451, manufactured by FUJIFILM Wako Pure Chemical Corporation) was added thereto, and the mixture was stirred using ROTATOR VMR-3R. This provided a 10 wt% 4VP-tBuMA stock solution. Separately, 1.0 g of PEGDGE1000(product number 805505, manufactured by Sigma-Aldrich Co. LLC) was added to a bottle, then 7.83 g of ethanol was added thereto, and the mixture was stirred using ROTATOR VMR-3R. This provided a 11.32 wt% stock solution of PEGDGE1000.

Thereafter, 49.98 mg of ethanol was added to the bottle, and then 106.02 mg of the PEGDGE1000 solution (0.6 wt%) prepared above was added, followed by 1144 mg of the tBuMA-4VP solution (5.72 wt%). Thereafter, the mixture was gently stirred with VORTEX-GENIE 2. After stirring, the opening of the bottle was sealed with parafilm and a polyimide tape, and the mixture was rotated and stirred in a thermostatic bath at 53°C for 16 hours. Thereafter, the bottle was taken out from the thermostatic bath, and the bottle taken out until immediately before the application was gently rotated and stirred with a tube rotator. Thus, a 4VP-tBuMA solution for coating was prepared.

### [3. Immersion experiment of sensor]

Thereafter, an immersion experiment of the sensor obtained in [2. Production of sensor containing synthesized polymer] was performed.

Specifically, the obtained sensor was immersed in 1.5 mL of pure water, and the sensor immersed in pure water was allowed to stand in a thermostatic bath at 37°C for 22 hours. Thereafter, the eluate from the sensor was collected, and the MAS concentration in the collected eluate was measured. The MAS concentration was measured using an ultraviolet-visible spectrophotometer (wavelength: 256 nm) (Model V-650 manufactured by JASCO Corporation).

### Comparative Example 3B (Example for Comparison with Example 3B)

Comparative Example 3B is different from Example 3B in that a phosphate buffer solution rather than a polymer was applied onto the working electrode in producing the sensor. The applied phosphate buffer solution was dried at room temperature after application.

In Comparative Example 3B, the concentration of the polymer solution applied onto the working electrode and the concentration of the phosphate buffer solution were set to be the same (1304.6 mmol/L), and the application amount was also set to be the same 1 µL. The kind and concentration of the constitution material of the protective membrane, which is a constituent of the sensor, were the same.

In Comparative Example 3B, the sensor immersion experiment after producing the sensor was performed in the same procedure as in Example 3B. Specifically, the obtained sensor was immersed in 1.5 mL of pure water, and the sensor immersed in pure water was allowed to stand in a thermostatic bath at 37°C for 22 hours.

Thereafter, the eluate from the sensor was collected, and the phosphoric acid concentration in the collected eluate was measured. The phosphoric acid concentration was measured using ion chromatography (Type ICS 5000+ manufactured by Thermo Scientific). Note that, from the viewpoint of avoiding overlapping with the description in Example 3B, the description of the overlapping part is omitted.

### [Measurement results]

In Comparative Example 3B, the phosphoric acid concentration in the eluate was 1.12 µmol and the elution rate was 85.81%, whereas in Example 3B, the MAS concentration in the eluate was 0.042 mg and the elution rate was 16.61%.

### [Evaluation]

From the above measurement results, it was found that in Example 3B, 84% of MAS constituting the polymer membrane could remain in the sensor covered with the protective membrane. On the other hand, in Comparative Example 3B, it was found that only 15% of phosphoric acid constituting the phosphate buffer membrane could remain in the sensor covered with the protective membrane.

That is, it was found that the elution of MAS to the outside through the protective membrane in Example 3B was suppressed about 5.2 times as compared with the elution of phosphoric acid to the outside through the protective membrane in Comparative Example 3B.

From the above, it was found that as compared with the case of using a phosphate buffer solution having a low molecular weight as in Comparative Example 3B, the polymer in Example 3B tends to remain in the polymer membrane (corresponding to the reagent layer in the present invention), and can suitably suppress the outflow to the culture medium side located outside the protective membrane.

Note that the present invention is not limited to the illustrated embodiments, and various improvements and design changes can be made without departing from the gist of the present invention.

An embodiment of the present invention (invention B) as described above includes the following preferred aspects.
<1B> A polymer including a proton accepting group and an ionic group as a repeating unit.
<2B> In <1B>, the polymer having water solubility.
<3B> In <1B> or <2B>, the polymer having a weight average molecular weight of 10,000 or more.
<4B> In any one of <1B> to <3B>, the polymer, wherein the ionic groups are of zwitterionic type.
<5B> In any one of <1B> to <4B>, the polymer, wherein the ionic group includes a quaternary ammonium group in a cationic state and a sulfo group or a carboxy group in an anionic state.
<6B> In any one of <1B> to <5B>, the polymer, wherein the proton accepting group is a heterocyclic nitrogen group.
<7B> In <6B>, the polymer, wherein the heterocyclic nitrogen group has a pKa of 4 to 8.
<8B> In <6B> or <7B>, the polymer, wherein the heterocyclic nitrogen group is at least one selected from the group consisting of a pyridyl group, an imidazole group, a benzimidazole group, and an isoquinolyl group.
<9B> In any one of <1B> to <8B>, the polymer, wherein the first moiety having an ionic group contains 3-[[2-(methacryloyloxy)ethyl]dimethylammonio]propane-1-sulfonate.
<10B> In any one of <1B> to <9B>, the polymer, wherein the second moiety having a proton accepting group contains 4-vinylpyridine.
<11B> In any of <1B> to <10B>, the polymer, wherein the repeating unit includes a moiety derived from 4-vinylpyridine and a moiety derived from 3-[[2-(methacryloyloxy)ethyl]dimethylammonio]propane-1-sulfonate.
<12B> A reagent including the polymer according to any one of <1B> to <11B> and an oxidoreductase that oxidizes or dehydrogenates the analyte.
<13B> A biosensor including a reagent layer including the reagent of <12B>.
<14B> In <13B>, the biosensor wherein the biosensor is a lactic acid sensor.
<15B> A method for synthesizing a polymer, the method including:
   a first step of preparing a first solution containing a proton accepting group;
   a second step of preparing a second solution containing an ionic group;
   a third step of adding the second solution to the first solution and mixing the first solution with the second solution; and
   a fourth step of performing a polymerization reaction using a mixture containing the first solution and the second solution in the presence of a polymerization initiator.
<16B> In <15B>, the method for synthesizing a polymer, wherein in the first step, the first solution containing a heterocyclic nitrogen group as the proton accepting group is prepared.
<17B> In <15B> or <16B>, the method for synthesizing a polymer, wherein the heterocyclic nitrogen group has a pKa of 4 to 8.
<18B> In <16B> or <17B>, the method for synthesizing a polymer, wherein the heterocyclic nitrogen group is at least one selected from the group consisting of a pyridyl group, an imidazole group, a benzimidazole group, and an isoquinolyl group.
<19B> In any one of <15B> to <18B>, the method for synthesizing a polymer, wherein the first solution is a solution containing 4-vinylpyridine.
<20B> In any one of <15B> to <19B>, the method for synthesizing a polymer, wherein the second solution in which the ionic group is a zwitterionic type is prepared in the second step.
<21B> In any one of <15B> to <20B>, the method for synthesizing a polymer, wherein the ionic group in the second solution contains a quaternary ammonium group in a cationic state and a sulfo group or a carboxy group in an anionic state.
<22B> In any one of <15B> to <21B>, the method for synthesizing a polymer, wherein the second solution is a solution containing 3-[[2-(methacryloyloxy)ethyl]dimethylammonio]propane-1-sulfonate.
<23B> In any one of <15B> to <22B>, the method for synthesizing a polymer, wherein in the second step, the substance containing an ionic group is dissolved in water as a solvent for 3 seconds or more and 7 seconds or less to prepare the second solution.
<24B> In any one of <15B> to <23B>, the method for synthesizing a polymer, wherein in the third step, the second solution prepared in the second step is added to the first solution within 0.5 seconds or more and 4 seconds or less.

### INDUSTRIAL APPLICABILITY

A biosensor including a reagent layer containing a polymer according to an embodiment of the present invention can suitably measure an analyte.

### REFERENCE SIGNS LIST

- 10: Reagent layer
- 20: Protective membrane
- 100: Biosensor
- A: Polymer
- B: Enzyme

## Claims

1. A reagent layer comprising a polymer containing a proton accepting group in a repeating unit and having a pH buffering ability, and an oxidoreductase that oxidizes or dehydrogenates an analyte.

2. The reagent layer according to claim 1, wherein the polymer has a buffering ability in a pH range in which the oxidoreductase can maintain activity.

3. The reagent layer according to claim 1 or 2, wherein the proton accepting group is a heterocyclic nitrogen group.

4. The reagent layer according to claim 1 or 2, wherein the proton accepting group is at least one selected from the group consisting of a phosphate group, a sulfo group, and a carboxy group in an ionization state.

5. The reagent layer according to claim 3, wherein the heterocyclic nitrogen group is at least one selected from the group consisting of an imidazole group, a pyridyl group, an indolyl group, a quinolyl group, an isoquinolyl group, a tetrahydroquinolyl group, a thiazole group, an indolidyl group, an imidazopyridyl group, an acridinyl group, a tetrazole group, a triazole group, a pyrazyl group, a morpholyl group, and a piperazyl group.

6. The reagent layer according to claim 1, wherein the polymer is polyvinylimidazole.

7. The reagent layer according to claim 1, wherein the polymer is poly-L-histidine.

8. The reagent layer according to claim 1, wherein the polymer is sodium polyphosphate.

9. The reagent layer according to claim 1, wherein the polymer has a weight average molecular weight of 10,000 or more.

10. The reagent layer according to claim 1, wherein the polymer comprises a moiety derived from 4-vinylpyridine and a moiety derived from 3-[[2-(methacryloyloxy)ethyl]dimethylammonio]propane-1 sulfonate as the repeating unit.

11. A biosensor comprising the reagent layer according to claim 1.

12. The biosensor according to claim 11, wherein the biosensor is a lactic acid sensor.

## Amended claims

### Amended claims under Art. 19.1 PCT

1. A reagent layer for an electrochemical sensor, comprising: a polymer having a proton accepting group in a repeating unit and having a buffering ability in a pH range of 6 to 8 and a weight average molecular weight of 10,000 or more and 1000000 or less; and an oxidoreductase capable of maintaining activity in the pH range for oxidizing or dehydrogenating an analyte.

3. The reagent layer for an electrochemical sensor according to claim 1, wherein the proton accepting group is a heterocyclic nitrogen group.

4. The reagent layer for an electrochemical sensor according to claim 1, wherein the proton accepting group is at least one selected from the group consisting of a phosphate group, a sulfo group, and a carboxy group in an ionization state.

5. The reagent layer for an electrochemical sensor according to claim 3, wherein the heterocyclic nitrogen group is at least one selected from the group consisting of an imidazole group, a pyridyl group, an indolyl group, a quinolyl group, an isoquinolyl group, a tetrahydroquinolyl group, a thiazole group, an indolidyl group, an imidazopyridyl group, an acridinyl group, a tetrazole group, a triazole group, a pyrazyl group, a morpholyl group, and a piperazyl group.

6. The reagent layer for an electrochemical sensor according to claim 1, wherein the polymer is polyvinylimidazole.

7. The reagent layer for an electrochemical sensor according to claim 1, wherein the polymer is poly-L-histidine.

8. The reagent layer for an electrochemical sensor according to claim 1, wherein the polymer is sodium polyphosphate.

10. The reagent layer for an electrochemical sensor according to claim 1, wherein the polymer comprises a moiety derived from 4-vinylpyridine and a moiety derived from 3-[[2-(methacryloyloxy)ethyl]dimethylammonio]propane-1 sulfonate as the repeating unit.

11. An electrochemical sensor for detecting or quantifying an analyte, comprising:
a working electrode; a counter electrode; the reagent layer according to claim 1 disposed on the working electrode; and a protective membrane, wherein the polymer does not substantially flow out.

12. The electrochemical sensor according to claim 11, wherein the electrochemical sensor is a lactic acid sensor.
